# EUROPEAN PATENT APPLICATION

(11) **EP 4 772 623 A2**
(43) Date of publication of application: **08.07.2026**
(21) Application number: 26170927.3
(22) Date of filing: 06.10.2021
(51) Int. Cl.: C12N 9/24

(54) **METHODS AND MATERIALS FOR TREATING GASTROINTESTINAL DISORDERS**

(30) Priority: 06.10.2020 US 202063087981 P
(62) Divisional of application: 21878471.8
(71) Applicant: Mayo Foundation for Medical Education and Research, Rochester, MN 55905 (US)
(72) Inventor: GROVER, Madhusudan, Rochester, Minnesota, 55902-1556 (US)
(74) Representative: Fish & Richardson P.C.

(57) **Abstract**

This document relates to methods and materials involved in treating a mammal having a gastrointestinal disorder (e.g., an irritable bowel syndrome (IBS) such as post-infection IBS (PI-IBS)). For example, one or more protease inhibitors and/or one or more microorganisms that produce one or more protease inhibitors can be administered to a mammal having, or at risk of developing, a gastrointestinal disorder to treat the mammal.

## Description

### CROSS-REFERENCE TO RELATED APPLICATIONS

This application claims the benefit of U.S. Patent Application Serial No. 63/087,981, filed on October 6, 2020. The disclosure of the prior application is considered part of (and is incorporated by reference in) the disclosure of this application.

### BACKGROUND

### 1. Technical Field

This document relates to methods and materials involved in treating a mammal having a gastrointestinal disorder (*e.g*., an irritable bowel syndrome (IBS) such as post-infection IBS (PI-IBS)). For example, one or more protease inhibitors and/or one or more microorganisms that can produce one or more protease inhibitors can be administered to a mammal having, or at risk of developing, a gastrointestinal disorder to treat the mammal.

### 2. Background Information

Irritable bowel syndrome (IBS) affects up to 15% of the U.S. population and results in significant morbidity and health care utilization. About 48 million U.S. adults suffer from infectious enteritis annually. These individuals have up to a four-fold increased risk of the intestinal infection developing into IBS, an entity characterized as PI-IBS.

Celiac disease affects 1% of the U.S. population and IBD affects approximately 0.5% of the U.S. population.

### SUMMARY

This document provides methods and materials for treating a mammal (e.g., a human) having a gastrointestinal disorder (*e.g*., an IBS such as PI-IBS). For example, this document provides methods and materials for using one or more protease inhibitors to treat a mammal having, or at risk of developing, a gastrointestinal disorder such as IBS (*e.g.,* PI-IBS). In some cases, a mammal having, or at risk of developing, a gastrointestinal disorder such as IBS (*e.g.,* PI-IBS) can be administered one or more protease inhibitors and/or one or more microorganisms that can produce (*e.g*., can produce and secrete) one or more protease inhibitors to treat the mammal.

The intestinal tract contains hundreds of host, microbial and dietary proteases that may play a role in host physiology including digestion and defense responses. As demonstrated herein, PI-IBS patients have high fecal proteolytic activity (PA) that is characterized by increased proteases (*e.g*., chymotrypsin like pancreatic elastase 2A, chymotrypsin like pancreatic elastase 3B, and trypsin-2) that are of host pancreatic origin and an absence of *Alistipes putredinis* in the intestinal microbiome. As also demonstrated herein, transfer of a community containing *A. putredinis* into high PA mice suppresses PA.

IBS is a chronic condition that typically needs to be managed long term. Having the ability to reduce or eliminate one or more symptoms of a gastrointestinal disorder such as IBS (*e.g.,* PI-IBS) can provide a unique and unrealized opportunity to treat mammals having a gastrointestinal disorder such as IBS (*e.g.,* PI-IBS). For example, a mammal having, or at risk of developing, a gastrointestinal disorder such as IBS (*e.g.,* PI-IBS) can be treated by administering one or more protease inhibitors to the mammal. In some cases, a mammal having, or at risk of developing, a gastrointestinal disorder such as IBS (e.g., PI-IBS) can be treated by administering one or more microorganisms that produce one or more protease inhibitors to the mammal.

In general, one aspect of this document features nutritional supplements that include a microorganism selected from the group consisting of *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis,* and combinations thereof. The microorganism can be encapsulated to be released in the intestine of a mammal. The nutritional supplement can be a liquid, a tablet, a capsule, a pill, a powder, a gel, or granules. The protease inhibitor can be a serine protease inhibitor. The protease inhibitor can be 4-benzenesulfonyl fluoride hydrochloride (AEBSF), nafamostat, secretory leucocyte protease inhibitor (SLPI), serpins, elafin, a host metabolite, a bacterial metabolite, or any combinations thereof. The protease inhibitor can inhibit the activity of a protease selected from the group consisting of trypsin-1, trypsin-2, trypsin-3, chymotrypsin like elastase 2A, chymotrypsin like elastase 3B, chymotrypsin, a kallikrein, plasmin, thrombin, and combinations thereof.

In another aspect, this document features food products including a microorganism having nucleic acid encoding a protease inhibitor. The microorganism can be *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis,* or any combinations thereof. The product can contain between 10² colony forming units (CFU) to about 10¹⁰ CFU of said microorganism. The food product can be yogurt, kefir, buttermilk, cheese, milk, milk powder, tea, juice, cookies, wafers, crackers, or cereals. The food product can include *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii,* and *Roseburia hominis.* The protease inhibitor can be a serine protease inhibitor. The protease inhibitor can be AEBSF, nafamostat, SLPI, serpins, elafin, a host metabolite, a bacterial metabolite, or any combinations thereof. The protease inhibitor can inhibit the activity of a protease selected from the group consisting of trypsin-1, trypsin-2, trypsin-3, chymotrypsin like elastase 2A, chymotrypsin like elastase 3B, chymotrypsin, a kallikrein, plasmin, thrombin, and combinations thereof.

In another aspect, this document features compositions that include a microorganism having an exogenous nucleic acid encoding a protease inhibitor. The microorganism can be *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis,* or any combinations thereof. The composition can include *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii,* and *Roseburia hominis.* The protease inhibitor can be a serine protease inhibitor. The protease inhibitor can be AEBSF, nafamostat, SLPI, serpins, elafin, a host metabolite, a bacterial metabolite, or any combinations thereof. The protease inhibitor can inhibit the activity of a protease selected from the group consisting of trypsin-1, trypsin-2, trypsin-3, chymotrypsin like elastase 2A, chymotrypsin like elastase 3B, chymotrypsin, a kallikrein, plasmin, thrombin, and combinations thereof. The composition can be formulated for oral administration. The composition can bea liquid, a tablet, a capsule, a pill, a powder, a gel, or granules.

In another aspect, this document features compositions that include an exogenous nucleic acid encoding a glucuronidase polypeptide. The microorganism can be *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* or *Bacteroides ovatus.* The composition can include *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.* The glucuronidase polypeptide can be a beta-glucuronidase polypeptide. The glucuronidase polypeptide can convert conjugated bilirubin to unconjugated bilirubin. The composition can be formulated for oral administration. The composition can be a liquid, a tablet, a capsule, a pill, a powder, a gel, or granules.

In another aspect, this document features methods for treating a mammal having IBS. The methods can include, or consist essentially of, administering a composition including a protease inhibitor to a mammal having IBS. The method can include identifying the mammal as having IBS. The mammal can be a human. The IBS can be PI-IBS. The administering of the composition to the mammal can be effective to reduce or eliminate a symptom of IBS in the mammal. The symptom of IBS can be abdominal pain, abdominal cramping, abdominal bloating, excess gas, diarrhea constipation, alternating bouts of diarrhea and constipation, weight loss, rectal bleeding, iron deficiency anemia, unexplained vomiting, difficulty swallowing, or any combinations thereof. The administering of the composition to the mammal can be effective to reduce intestinal permeability in the gastrointestinal tract of the mammal. The administering of the composition to the mammal can be effective to reduce a level of proteolytic activity of a protease in the gastrointestinal tract of the mammal. The protease can be trypsin-1, trypsin-2, trypsin-3, chymotrypsin like elastase 2A, chymotrypsin like elastase 3B, chymotrypsin, a kallikrein, plasmin, thrombin, or any combinations thereof. The level of proteolytic activity of the protease can be measured using zyomgraphy. The administering of the composition to the mammal can be effective to increase the level of a protease inhibitor in the gastrointestinal tract of the mammal. The protease inhibitor can be AEBSF, nafamostat, SLPI, serpins, elafin, a host metabolite, a bacterial metabolite, or any combinations thereof.

In another aspect, this document features methods for treating a mammal having IBS. The methods can include, or consist essentially of, administering a composition including a microorganism containing nucleic acid encoding a protease inhibitor to a mammal having IBS. The method can include identifying the mammal as having IBS. The mammal can be a human. The IBS can be PI-IBS. The administering of the composition to the mammal can be effective to reduce or eliminate a symptom of IBS in the mammal. The symptom of IBS can be abdominal pain, abdominal cramping, abdominal bloating, excess gas, diarrhea constipation, alternating bouts of diarrhea and constipation, weight loss, rectal bleeding, iron deficiency anemia, unexplained vomiting, difficulty swallowing, or any combinations thereof. The administering of the composition to the mammal can be effective to reduce intestinal permeability in the gastrointestinal tract of the mammal. The administering of the composition to the mammal can be effective to reduce a level of proteolytic activity of a protease in the gastrointestinal tract of the mammal. The protease can be trypsin-1, trypsin-2, trypsin-3, chymotrypsin like elastase 2A, chymotrypsin like elastase 3B, chymotrypsin, a kallikrein, plasmin, thrombin, or any combinations thereof. The level of proteolytic activity of the protease can be measured using zyomgraphy. The administering of the composition to the mammal can be effective to increase the level of a protease inhibitor in the gastrointestinal tract of the mammal. The protease inhibitor can be AEBSF, nafamostat, SLPI, serpins, elafin, a host metabolite, a bacterial metabolite, or any combinations thereof. The microorganism can be *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis,* or any combinations thereof.

In another aspect, this document features methods for treating a mammal having IBS. The methods can include, or consist essentially of, administering a composition including a microorganism containing nucleic acid encoding a protease inhibitor and/or nucleic acid encoding a glucuronidase (e.g., a beta-glucuronidase) polypeptide to a mammal having IBS. The method can include identifying the mammal as having IBS. The mammal can be a human. The IBS can be PI-IBS. The administering of the composition to the mammal can be effective to reduce or eliminate a symptom of IBS in the mammal. The symptom of IBS can be abdominal pain, abdominal cramping, abdominal bloating, excess gas, diarrhea constipation, alternating bouts of diarrhea and constipation, weight loss, rectal bleeding, iron deficiency anemia, unexplained vomiting, difficulty swallowing, or any combinations thereof. The administering of the composition to the mammal can be effective to reduce intestinal permeability in the gastrointestinal tract of the mammal. The administering of the composition to the mammal can be effective to reduce a level of proteolytic activity of a protease in the gastrointestinal tract of the mammal. The protease can be trypsin-1, trypsin-2, trypsin-3, chymotrypsin like elastase 2A, chymotrypsin like elastase 3B, chymotrypsin, a kallikrein, plasmin, thrombin, or any combinations thereof. The level of proteolytic activity of the protease can be measured using zyomgraphy. The administering of the composition to the mammal can be effective to increase the level of a protease inhibitor in the gastrointestinal tract of the mammal. The protease inhibitor can be AEBSF, nafamostat, SLPI, serpins, elafin, a host metabolite, a bacterial metabolite, or any combinations thereof. The microorganism can be *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis,* or any combinations thereof. The composition can include a microorganism containing nucleic acid encoding a protease inhibitor. The composition can include a microorganism containing nucleic acid encoding a glucuronidase (e.g., a beta-glucuronidase) polypeptide. The composition can include a microorganism containing nucleic acid encoding a protease inhibitor and nucleic acid encoding a glucuronidase (e.g., a beta-glucuronidase) polypeptide.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

Figure 1: Schematic of an exemplary role of serine proteases in pathophysiology of PI-IBS.
Figure 2: Distribution of high PA and low PA patients in PI-IBS vs. healthy volunteers (p<0.05, F-exact).
Figure 3: Volcano plot showing abundance of fecal proteins in high PA compared to low PA supernatants. Three proteins (all human) had *FDR<0.05.* n=7 high and 6 low PA subjects. Solid circles human, and hollow circles microbial proteins. -Log₁₀(*FDR* 0.05)=1.3.
Figure 4: No significant differences between colonic mucosal expressions of common proteases (corrected *p>0.05* for all).
Figure 5: *In situ* zymography showing no difference in colonic tissue PA (Mann Whitney, n=3-4/group).
Figure 6: High PA PI-IBS and low PA have differences in microbiota composition. PERMANOVA *p*<0.05. n=12 high PA and 21 healthy volunteers.
Figure 7: Mice humanized with high PA PI-IBS feces have greater PA in humanized state than mice humanized with healthy volunteer (low PA) feces (*p*=0.07, generalized linear model for group differences). n=6 high and 6 low PA patients and 7-8 humanized mice/patient.
Figure 8: Humanized mouse with high fecal PA have greater abundance of Trypsin 1 and 2 compared to mouse with low fecal PA.
Figure 9: PA drops in cecum in conventional mouse, low PA humanized mouse but not high PA humanized mouse. n=1 mouse/group.
Figure 10: Experimental plan for humanized mice with high PA PI-IBS and low PA (healthy volunteer) feces.
Figure 11: Metaproteomics and bioinformatics workflow.
Figure 12: *A. putredinis* (%) in high and low PA patients. n=12/high PA, 21/low PA. *p*<0.01, Mann-Whitney test.
Figure 13: Abundance of I87 inhibitor in high PA and low PA patients. n=12/high PA, 21/low PA. *p*<0.01, Mann-Whitney test.
Figure 14: Fecal microbial transplant from low PA human stool to high PA humanized mice results in suppression of fecal PA. n=1 humanized state for donor and recipient and 9 mice/group.
Figures 15A - 15B: Experimental plan for *A. putredinis.* (Figure 15A) Effects of monocolonization in germ free (GF) mice. (Figure 15B) Effects of chronic administration in high PA PI-IBS humanized mice.
Figures 16A - 16B: High PA fecal supernatants cause (Figure 16A) TER drop and (Figure 16B) Texas red dextran flux across Caco-2 monolayers. Black tracing PBS. n=5/group, **p*<0.01, Mann Whitney.
Figures 17A - 17B: Fecal supernatant effects on (Figure 17A) TER and (Figure 17B) Texas red dextran flux across Caco-2 monolayers blocked by serine (AEBSF) but not cysteine (E64) protease inhibitor (n=5/group, **p*<0.01, Kruskal Wallis).
Figure 18: PAR-2 inhibitor blocks effect of high PA fecal supernatant on TER. n=5/group, **p*<0.05, Mann Whitney.
Figure 19: High PA supernatant cause MLC phosphorylation. **p*<0.05, Mann Whitney.
Fig. 20: High PA supernatants decrease occludin protein expression. **p*<0.05, Mann Whitney.
Figures 21A - 21B: High PA patients have higher *in vivo* colonic permeability than low PA patients as measured by (Figure 21A) 2-24 hour lactulose excretion and (Figure 21B) 2-24 hour lactulose/13C mannitol excretion ratio. **p*<0.01, Mann Whitney.
Figures 22A - 22B: High PA humanized mice have greater *in vivo* permeability of orally gavaged (Figure 22A) creatinine (**p*<0.05) and (Figure 22B) FITC-Dextran (**p*<0.05). Generalized linear models for group differences. n=6 high and 6 low PA patients and 3 humanized mice/patient.
Figures 23A - 23B: Colonoid monolayer (Figure 23A) TER tracing (colonoid; blank well) and (Figure 23B) Occludin immunofluorescence.

### DETAILED DESCRIPTION

This document provides methods and materials for treating a mammal (*e.g.,* a human) having a gastrointestinal disorder (*e.g.,* an IBS such as PI-IBS). For example, this document provides methods and materials for using one or more protease inhibitors (and/or one or more microorganisms that produce one or more protease inhibitors) to treat a mammal having, or at risk of developing, a gastrointestinal disorder such as IBS (*e.g.,* PI-IBS). In some cases, a mammal having a gastrointestinal disorder such as IBS (*e.g.,* PI-IBS) can be administered one or more protease inhibitors (*e.g.,* a composition including one or more protease inhibitors) to treat the mammal. In some cases, a mammal having a gastrointestinal disorder such as IBS (*e.g.,* PI-IBS) can be administered one or more microorganisms that produce (*e.g.,* that produce and secrete one or more protease inhibitors) such as a composition that includes one or more microorganisms that produce one or more protease inhibitors to treat the mammal.

Any appropriate protease inhibitor can be used to treat a mammal having, or at risk of developing, a gastrointestinal disorder such as IBS (*e.g.,* PI-IBS) as described herein (*e.g.,* by administering a composition that includes one or more protease inhibitors and/or one or more microorganisms that produce one or more protease inhibitors to the mammal). A protease inhibitor can be an inhibitor of protease activity or an inhibitor of protease expression. A protease inhibitor can be any type of molecule (e.g., a polypeptide, a nucleic acid, or a small molecule). In some cases, a protease inhibitor can be a metabolite (e.g., a host metabolite or a bacterial metabolite). Examples of compounds that can reduce protease activity include, without limitation, 4-benzenesulfonyl fluoride hydrochloride (AEBSF), nafamostat, secretory leucocyte protease inhibitor (SLPI), serpins, elafin, miropins, and unconjugated bilirubin. Examples of compounds that can reduce protease expression and be used as described herein include, without limitation, nucleic acid molecules designed to induce RNA interference against protease expression (e.g., a small interfering RNA (siRNA) molecule or a short hairpin RNA (shRNA) molecule), antisense molecules against protease expression, and miRNAs against protease expression. In some cases, a protease inhibitor can be an I87 protease inhibitor. In some cases, a protease inhibitor can be a serine protease inhibitor. In some cases, a protease inhibitor is a protease inhibitor that is not a cysteine protease inhibitor.

A protease inhibitor can target (*e.g.,* can inhibit) any appropriate protease(s). Examples of proteases that can be targeted by a protease inhibitor described herein include, without limitation, trypsins (*e.g.,* trypsin-1, trypsin-2, and trypsin-3), chymotrypsin like elastases (*e.g.,* chymotrypsin like elastase 2A and chymotrypsin like elastase 3B), chymotrypsin, kallikreins (*e.g.,* plasma kallikrein, tissue kallikrein-related peptidases), plasmin, thrombin, neutrophilic elastases, and matrix metalloproteases. In some cases, a protease that can be targeted by a protease inhibitor described herein can be a serine protease. In some cases, a protease that can be targeted by a protease inhibitor described herein can be a pancreatic protease.

Any appropriate microorganism (*e.g.,* live microorganism) that produces (*e.g.,* produces and secretes) one or more protease inhibitors can be used to treat a mammal having, or at risk of developing, a gastrointestinal disorder such as IBS (*e.g.,* PI-IBS) as described herein (*e.g.,* by administering a composition containing one or more microorganisms that produce one or more protease inhibitors to the mammal). Such a microorganism can be any type of microorganism (*e.g.,* bacteria, viruses, or fungi). Examples of microorganisms that can produce one or more protease inhibitors include, without limitation, *A. putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.* In some cases, a microorganism can include one or more endogenous nucleic acids that encode a protease inhibitor. In some cases, a microorganism can include (*e.g.,* can be engineered to include) exogenous nucleic acid encoding a protease inhibitor. For example, a microorganism can include recombinant and/or transgenic nucleic acid that encodes a protease inhibitor. In some cases, a microorganism can include one or more endogenous nucleic acids that encode a polypeptide that can catalyze formation of a protease inhibitor. In some cases, a microorganism can include (e.g., can be engineered to include) exogenous nucleic acid encoding a polypeptide that can catalyze formation of a protease inhibitor. For example, a microorganism can include recombinant and/or transgenic nucleic acid that encodes a beta-glucuronidase (GUSB) polypeptide (e.g., thereby converting conjugated bilirubin into unconjugated bilirubin, for example, as described in the Examples).

When a microorganism includes exogenous nucleic acid encoding a protease inhibitor, the exogenous nucleic acid can encode any appropriate protease inhibitor (e.g., SLPI, a serpin, and an elafin). Examples of protease inhibitors and nucleic acids encoding protease inhibitors include, without limitation, those set forth in the National Center for Biotechnology Information (NCBI) databases at, for example, accession no. NM_011414 (version no. NM_011414.3), accession no. NM_003064 (version no. NM_003064.4), accession no. AM402969 (version no. AM402969.1), and accession no. L10343 (version no. L10343.1).

When a microorganism includes exogenous nucleic acid encoding a glucuronidase polypeptide (e.g., a beta-glucuronidase polypeptide), the exogenous nucleic acid can encode any appropriate glucuronidase polypeptide. Examples of glucuronidase polypeptides and nucleic acids encoding glucuronidase polypeptides include, without limitation, those set forth in the NCBI databases at, for example, accession no. M19279 (version no. M19279.1), accession no. J02836 (version no. J02836.1), accession no. NM_000181 (version no. NM_000181.4), accession no. NM_001284290 (version no. NM_001284290.2), accession no. NM_001293104 (version no. NM_001293104.2), and accession no. NM_001293105 (version no. NM_001293105.2).

In addition to nucleic acid encoding a protease inhibitor (e.g., SLPI, a serpin, and an elafin) and/or nucleic acid encoding a glucuronidase polypeptide (e.g., a beta-glucuronidase polypeptide), an exogenous nucleic acid also can include one or more regulatory elements operably linked to the nucleic acid encoding the protease inhibitor and/or nucleic acid encoding the glucuronidase polypeptide. Such regulatory elements can include promoter sequences, enhancer sequences, response elements, signal peptides, internal ribosome entry sequences, polyadenylation signals, terminators, and inducible elements that modulate expression (*e.g.,* transcription or translation) of a nucleic acid. The choice of regulatory element(s) can depend on several factors, including, without limitation, inducibility, targeting, and the level of expression desired. For example, a promoter can be included in an exogenous nucleic acid encoding a protease inhibitor (e.g., SLPI, a serpin, and an elafin) to facilitate transcription of a nucleic acid encoding the protease inhibitor. For example, a promoter can be included in an exogenous nucleic acid encoding a glucuronidase polypeptide (e.g., a beta-glucuronidase polypeptide) to facilitate transcription of a nucleic acid encoding the glucuronidase polypeptide. A promoter can be a naturally occurring promoter or a recombinant promoter. A promoter can be a strong promoter (e.g., can promote a high rate of transcription) or a weak promoter. A promoter can be ubiquitous or inducible (*e.g.,* in the presence of tetracycline), and can affect the expression of a nucleic acid encoding a protease inhibitor (e.g., SLPI, a serpin, and an elafin) and/or a nucleic acid encoding a glucuronidase polypeptide (e.g., a beta-glucuronidase polypeptide) in a general or tissue-specific manner. Examples of promoters that can be used to drive expression of a protease inhibitor (e.g., SLPI, a serpin, and an elafin) and/or a glucuronidase polypeptide (e.g., a beta-glucuronidase polypeptide) in microorganisms include, without limitation, recA promoters, araBAD promoters, T7 promoters, Sp6 promoters, lac promoters, trp promoters, and Ptac promoters. As used herein, "operably linked" refers to positioning of a regulatory element in an exogenous nucleic acid relative to a nucleic acid encoding a protease inhibitor (e.g., SLPI, a serpin, and an elafin) and/or a nucleic acid encoding a glucuronidase polypeptide (e.g., a beta-glucuronidase polypeptide) in such a way as to permit or facilitate expression of the encoded protease inhibitor and/or the encoded glucuronidase polypeptide.

Any appropriate mammal having, or at risk of developing, a gastrointestinal disorder (*e.g.,* an IBS such as PI-IBS) can be treated as described herein (*e.g.,* by administering one or more protease inhibitors and/or one or more microorganisms that produce one or more protease inhibitors to the mammal). Examples of mammals having, or at risk of developing, a gastrointestinal disorder that can be treated as described herein include, without limitation, humans, non-human primates (*e.g.,* monkeys), dogs, cats, horses, cows, pigs, sheep, mice, and rats. In some cases, a mammal having, or at risk of developing, a gastrointestinal disorder that can be treated as described herein can have a *C. jejuni* infection (*e.g.,* a culture positive *C. jejuni* infection). In some cases, a human having, or at risk of developing, a gastrointestinal disorder can be treated by administering one or more protease inhibitors and/or one or more microorganisms that produce one or more protease inhibitors (*e.g.,* a composition including one or more protease inhibitors and/or one or more microorganisms that produce one or more protease inhibitors) to the human.

Any appropriate gastrointestinal disorder can be treated as described herein (*e.g.,* by administering one or more protease inhibitors and/or one or more microorganisms that produce one or more protease inhibitors to the mammal). Examples of gastrointestinal disorders that can be treated as described herein include, without limitation, IBS (*e.g.,* PI-IBS), celiac disease, inflammatory bowel disease (IBD), infectious gastroenteritis (e.g., infectious gastroenteritis caused by, for example, *E. coli, Salmonella, Giardia, Shigella,* and/or *C*. *jejuni*), and pouchitis. In some cases, a mammal (*e.g.,* a human) having, or at risk of developing, PI-IBS can be treated by administering one or more protease inhibitors and/or one or more microorganisms that produce one or more protease inhibitors (*e.g.,* a composition including one or more protease inhibitors and/or one or more microorganisms that produce one or more protease inhibitors) to the mammal.

In some cases, methods for treating a mammal (*e.g.,* a human) having, or at risk of developing, a gastrointestinal disorder (*e.g.,* an IBS such as PI-IBS), also can include identifying a mammal as having, or as being at risk of developing, a gastrointestinal disorder. Any appropriate method can be used to identify a mammal as having, or as being at risk of developing, a gastrointestinal disorder. For example, physical examination, Rome criteria, Manning criteria, imaging tests (*e.g.,* flexible sigmoidoscopy, colonoscopy, upper endoscopy, X-ray, and computerized tomography (CT) scan), laboratory tests (*e.g.,* lactose intolerance tests, breath test for bacterial overgrowth, and stool tests), gastrointestinal transit, anorectal barostat, small bowel or fecal microbiome assessments, and/or gastrointestinal permeability can be used to identify a mammal as having, or as being at risk of developing, a gastrointestinal disorder (*e.g.,* an IBS such as PI-IBS). In some cases, identifying a mammal as having, or as being at risk of developing, a gastrointestinal disorder can include identifying the mammal as having a high level of PA *(e.g.,* high fecal PA). For example, detection of increased proteases (*e.g.,* chymotrypsin like pancreatic elastase 2A, chymotrypsin like pancreatic elastase 3B, and trypsin-2) in the gastrointestinal tract of a mammal (*e.g.,* as measured in a stool sample obtained from a mammal), substrate-based assays for proteolytic digestion (*e.g.,* casein substrate-based assays for proteolytic digestion), proteomics (e.g., tissue proteomics such as tissue activity based proteomics), transcriptomics (e.g., mRNA or transcriptomic expression in tissues), imaging (e.g., *in vivo* imaging with designed substrates), and/or tissue (e.g., intestinal tissue) zymography can be used to identify a mammal as having, or as being at risk of developing, a gastrointestinal disorder (*e.g.,* an IBS such as PI-IBS).

Once identified as having, or as being at risk of developing, a gastrointestinal disorder (*e.g.,* an IBS such as PI-IBS), the mammal *(e.g.,* the human) can be administered, or instructed to self-administer, one or more protease inhibitors and/or one or more microorganisms that produce one or more protease inhibitors (*e.g.,* a composition including one or more protease inhibitors and/or one or more microorganisms that produce one or more protease inhibitors).

In some cases, a mammal (*e.g.,* a human) having, or at risk of developing, a gastrointestinal disorder (*e.g.,* an IBS such as PI-IBS) can be administered one or more protease inhibitors and/or one or more microorganisms that produce one or more protease inhibitors (*e.g.,* a composition including one or more protease inhibitors and/or one or more microorganisms that produce one or more protease inhibitors) to reduce or eliminate one or more symptoms of a gastrointestinal disorder. Examples of symptoms of a gastrointestinal disorder that can be reduced or eliminated as described herein include, without limitation, abdominal pain, abdominal cramping, abdominal bloating, excess gas, diarrhea (*e.g.,* diarrhea at night), constipation, alternating bouts of diarrhea and constipation, weight loss, rectal bleeding, iron deficiency anemia, unexplained vomiting, difficulty swallowing, and dietary intolerances. For example, one or more protease inhibitors and/or one or more microorganisms that produce one or more protease inhibitors can be administered to a mammal (*e.g.,* a human) in need thereof (*e.g.,* a human having, or at risk of developing, a gastrointestinal disorder (*e.g.,* an IBS such as PI-IBS)) as described herein to reduce the severity of one or more symptoms of a gastrointestinal disorder by, for example, 10, 20, 30, 40, 50, 60, 70, 80, 90, 95, or more percent.

In some cases, a mammal (*e.g.,* a human) having, or at risk of developing, a gastrointestinal disorder (*e.g.,* an IBS such as PI-IBS) can be administered one or more protease inhibitors and/or one or more microorganisms that produce one or more protease inhibitors (*e.g.,* a composition including one or more protease inhibitors and/or one or more microorganisms that produce one or more protease inhibitors) to reduce or eliminate intestinal permeability (*e.g.,* leaky gut). For example, one or more protease inhibitors and/or one or more microorganisms that produce one or more protease inhibitors can be administered to a mammal (*e.g.,* a human) in need thereof (*e.g.,* a human having, or at risk of developing, a gastrointestinal disorder (*e.g.,* an IBS such as PI-IBS)) as described herein to reduce intestinal permeability in a mammal by, for example, 10, 20, 30, 40, 50, 60, 70, 80, 90, 95, or more percent.

In some cases, a mammal (*e.g.,* a human) having, or at risk of developing, a gastrointestinal disorder (*e.g.,* an IBS such as PI-IBS) can be administered one or more protease inhibitors and/or one or more microorganisms that produce one or more protease inhibitors (*e.g.,* a composition including one or more protease inhibitors and/or one or more microorganisms that produce one or more protease inhibitors) to reduce or eliminate intestinal inflammation. For example, one or more protease inhibitors and/or one or more microorganisms that produce one or more protease inhibitors can be administered to a mammal (*e.g.,* a human) in need thereof (*e.g.,* a human having, or at risk of developing, a gastrointestinal disorder (*e.g.,* an IBS such as PI-IBS)) as described herein to reduce intestinal inflammation in a mammal by, for example, 10, 20, 30, 40, 50, 60, 70, 80, 90, 95, or more percent.

In some cases, a mammal (*e.g.,* a human) having, or at risk of developing, a gastrointestinal disorder (*e.g.,* an IBS such as PI-IBS) can be administered one or more protease inhibitors and/or one or more microorganisms that produce one or more protease inhibitors (*e.g.,* a composition including one or more protease inhibitors and/or one or more microorganisms that produce one or more protease inhibitors) to reduce or eliminate visceral hypersensitivity. For example, one or more protease inhibitors and/or one or more microorganisms that produce one or more protease inhibitors can be administered to a mammal (*e.g.,* a human) in need thereof (*e.g.,* a human having, or at risk of developing, a gastrointestinal disorder (*e.g.,* an IBS such as PI-IBS)) as described herein to reduce visceral hypersensitivity in a mammal by, for example, 10, 20, 30, 40, 50, 60, 70, 80, 90, 95, or more percent.

In some cases, a mammal (*e.g.,* a human) having, or at risk of developing, a gastrointestinal disorder (*e.g.,* an IBS such as PI-IBS) can be administered one or more protease inhibitors and/or one or more microorganisms that produce one or more protease inhibitors (*e.g.,* a composition including one or more protease inhibitors and/or one or more microorganisms that produce one or more protease inhibitors) to modify (e.g., reduce) gastrointestinal transit time. For example, one or more protease inhibitors and/or one or more microorganisms that produce one or more protease inhibitors can be administered to a mammal (*e.g.,* a human) in need thereof (*e.g.,* a human having, or at risk of developing, a gastrointestinal disorder (*e.g.,* an IBS such as PI-IBS)) as described herein to reduce gastrointestinal transit in a mammal by, for example, 10, 20, 30, 40, 50, 60, 70, 80, 90, 95, or more percent.

In some cases, a mammal (*e.g.,* a human) having, or at risk of developing, a gastrointestinal disorder (*e.g.,* an IBS such as PI-IBS) can be administered one or more protease inhibitors and/or one or more microorganisms that produce one or more protease inhibitors (*e.g.,* a composition including one or more protease inhibitors and/or one or more microorganisms that produce one or more protease inhibitors) to reduce or eliminate the level of PA in the mammal (*e.g.,* in the mammal's gastrointestinal tract). The term "reduced level" as used herein with respect to PA refers to any level that is lower than a reference level of PA. The term "reference level" as used herein with respect to PA refers to the level of PA typically observed in the gastrointestinal tract of one or more mammals not having a gastrointestinal disorder (*e.g.,* one or more healthy mammals). In some cases, a reduced level of PA can be an undetectable level of PA. For example, one or more protease inhibitors and/or one or more microorganisms that produce one or more protease inhibitors can be administered to a mammal (*e.g.,* a human) in need thereof (*e.g.,* a human having, or at risk of developing, a gastrointestinal disorder (*e.g.,* an IBS such as PI-IBS)) as described herein to reduce the level of PA in the gastrointestinal tract of a mammal by, for example, 10, 20, 30, 40, 50, 60, 70, 80, 90, 95, or more percent. PA can be measured using any appropriate technique. In some cases, PA can be measured using detection of increased proteases (*e.g.,* chymotrypsin like pancreatic elastase 2A, chymotrypsin like pancreatic elastase 3B, and trypsin-2) in the gastrointestinal tract of a mammal (*e.g.,* as measured in a stool sample obtained from a mammal), substrate-based assays for proteolytic digestion (*e.g.,* casein substrate-based assays for proteolytic digestion), proteomics (e.g., tissue proteomics such as tissue activity based proteomics), and/or transcriptomics (e.g., mRNA or transcriptomic expression in tissues). In some cases, PA can be measured as described in Example 1 and/or in Example 3.

In some cases, a mammal (*e.g.,* a human) having, or at risk of developing, a gastrointestinal disorder (*e.g.,* an IBS such as PI-IBS) can be administered one or more protease inhibitors and/or one or more microorganisms that produce one or more protease inhibitors (*e.g.,* a composition including one or more protease inhibitors and/or one or more microorganisms that produce one or more protease inhibitors) to reduce or eliminate the level of one or more proteases in the mammal (*e.g.,* in the mammal's gastrointestinal tract). The term "reduced level" as used herein with respect to one or more proteases refers to any level that is lower than a reference level of the protease(s). The term "reference level" as used herein with respect to one or more proteases refers to the level of the protease(s) typically observed in the gastrointestinal tract of one or more mammals not having a gastrointestinal disorder (*e.g.,* one or more healthy mammals). In some cases, a reduced level of one or more proteases can be an undetectable level of the protease(s). For example, one or more protease inhibitors and/or one or more microorganisms that produce one or more protease inhibitors can be administered to a mammal (*e.g.,* a human) in need thereof (*e.g.,* a human having, or at risk of developing, a gastrointestinal disorder (*e.g.,* an IBS such as PI-IBS)) as described herein to reduce the level of one or more proteases in the gastrointestinal tract of a mammal by, for example, 10, 20, 30, 40, 50, 60, 70, 80, 90, 95, or more percent. A level of protease(s) can be measured using any appropriate technique. In some cases, a level of a protease can be measured using immunoassays (*e.g.,* immunohistochemistry (IHC) techniques and western blotting techniques), mass spectrometry techniques (*e.g.,* proteomics-based mass spectrometry assays), transmission electron microscopy, and/or enzyme-linked immunosorbent assays (ELISAs). In some cases, a level of a nucleic acid (*e.g.,* mRNA) encoding a protease can be measured using northern blotting techniques, *in situ* hybridization techniques (*e.g.,* fluorescent *in situ* hybridization), and/or reverse transcription-polymerase chain reaction (RT-PCR) techniques. In some cases, a level of a protease can be measured as described in Example 1.

In some cases, a mammal (*e.g.,* a human) having, or at risk of developing, a gastrointestinal disorder (*e.g.,* an IBS such as PI-IBS) can be administered one or more protease inhibitors and/or one or more microorganisms that produce one or more protease inhibitors (*e.g*., a composition including one or more protease inhibitors and/or one or more microorganisms that produce one or more protease inhibitors) to increase the level of one or more protease inhibitors in the mammal (*e.g.,* in the mammal's gastrointestinal tract). The term "increased level" as used herein with respect to one or more protease inhibitors refers to any level that is higher than a reference level of the protease inhibitor(s). The term "reference level" as used herein with respect to one or more protease inhibitors refers to the level of the protease inhibitor(s) typically observed in the gastrointestinal tract of one or more mammals not having a gastrointestinal disorder (*e.g*., one or more healthy mammals). For example, one or more protease inhibitors and/or one or more microorganisms that produce one or more protease inhibitors can be administered to a mammal (*e.g*., a human) in need thereof (*e.g.,* a human having, or at risk of developing, a gastrointestinal disorder (*e.g.,* an IBS such as PI-IBS)) as described herein to increase the level of one or more protease inhibitors in the gastrointestinal tract of a mammal by, for example, 10, 20, 30, 40, 50, 60, 70, 80, 90, 95, or more percent. A level of protease inhibitor(s) can be measured using any appropriate technique. In some cases, a level of a protease inhibitor can be measured using immunoassays (*e.g.,* IHC techniques and western blotting techniques), mass spectrometry techniques (*e.g.,* proteomics-based mass spectrometry assays), transmission electron microscopy, and/or ELISAs. In some cases, a level of a nucleic acid (*e.g.,* mRNA) encoding a protease inhibitor and/or a nucleic acid encoding a glucuronidase (e.g., a beta-glucuronidase) polypeptide can be measured using northern blotting techniques, *in situ* hybridization techniques (*e.g.,* fluorescent *in situ* hybridization), and/or RT-PCR techniques. In some cases, a level of a protease inhibitor can be measured as described in Example 1.

In some cases, a mammal (*e.g.,* a human) having, or at risk of developing, a gastrointestinal disorder (*e.g.,* an IBS such as PI-IBS) can be administered one or more protease inhibitors and/or one or more microorganisms that produce one or more protease inhibitors (*e.g.,* a composition including one or more protease inhibitors and/or one or more microorganisms that produce one or more protease inhibitors) to alter the microbiome within a mammal (*e.g.,* within the gastrointestinal tract of a mammal). In some cases, a microbiome can be altered to increase the level of one or more microorganisms that produce one or more protease inhibitors within the gastrointestinal tract of a mammal. Examples of microorganisms that can produce one or more protease inhibitors and can be increased within the gastrointestinal tract of a mammal as described herein include, without limitation, A. *putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.* The term "increased level" as used herein with respect to one or more microorganisms that produce one or more protease inhibitors refers to any level that is higher than a reference level of the microorganism(s) that can produce one or more protease inhibitors. The term "reference level" as used herein with respect to one or more microorganisms that produce one or more protease inhibitors refers to the level of the microorganism(s) that can produce one or more protease inhibitors typically observed in the gastrointestinal tract of one or more mammals not having a gastrointestinal disorder (*e.g.,* one or more healthy mammals). For example, one or more microorganisms that produce one or more protease inhibitors can be administered to a mammal (*e.g.,* a human) in need thereof *(e.g.,* a human having, or at risk of developing, a gastrointestinal disorder (*e.g.,* an IBS such as PI-IBS)) as described herein to increase the level of microorganisms that produce one or more protease inhibitors by, for example, 10, 20, 30, 40, 50, 60, 70, 80, 90, 95, or more percent. In cases where *A. putredinis* that produce one or more protease inhibitors are administered to a mammal in need thereof as described herein, the level of *A. putredinis* in the gastrointentisinal tract of the mammal can be increased by, for example, 10, 20, 30, 40, 50, 60, 70, 80, 90, 95, or more percent. In cases where *A. putredinis* that produce one or more protease inhibitors are administered to a mammal in need thereof as described herein, the level of *A. putredinis* in the gastrointentisinal tract of the mammal can be increased to account for from about 1% to about 15% (e.g., from about 1% to about 12%, from about 1% to about 10%, from about 1% to about 8%, from about 1% to about 5%, from about 3% to about 15%, from about 5% to about 15%, from about 7% to about 15%, from about 11% to about 15%, from about 13% to about 15%, from about 2% to about 12%, from about 5% to about 10%, from about 3% to about 5%, from about 5% to about 8%, or from about 8% to about 12%) of the microbiota in the gastrointensintal tract of the mammal. A level of a microorganism that produces one or more protease inhibitors can be measured using any appropriate technique. In some cases, a level of a microorganism that produces one or more protease inhibitors can be measured as described in Example 2.

In some cases, one or more protease inhibitors and/or one or more microorganisms that produce one or more protease inhibitors can be formulated into a composition (*e.g.,* a pharmaceutically acceptable composition) for administration to a mammal having, or at risk of developing, a gastrointestinal disorder (*e.g.,* an IBS such as PI-IBS). For example, one or more protease inhibitors and/or one or more microorganisms that produce one or more protease inhibitors can be formulated together with one or more other ingredients such as buffers, radical scavengers, antioxidants, reducing agents, or mixtures thereof. In some cases, a composition containing one or more protease inhibitors and/or one or more microorganisms that produce one or more protease inhibitors can be formulated to contain botanicals, vitamins, minerals, or combinations thereof. For example, one or more protease inhibitors and/or one or more microorganisms that produce one or more protease inhibitors can be formulated together with one or more pharmaceutically acceptable carriers (additives), excipients, and/or diluents including, without limitation, sterile aqueous or non-aqueous solutions, suspensions, and emulsions. Examples of non-aqueous solvents include, without limitation, propylene glycol, polyethylene glycol, vegetable oils, and organic esters. Aqueous carriers include, without limitation, water, alcohol, saline, and buffered solutions. Pharmaceutically acceptable carriers also can include physiologically acceptable aqueous vehicles (e.g., physiological saline) or other known carriers for oral administration. Examples of pharmaceutically acceptable carriers, excipients, and diluents that can be used in a composition described herein include, without limitation, phosphate buffered saline (PBS), inulin, dextrin, sucrose, lactose, starch (*e.g.,* starch glycolate), cellulose, cellulose derivatives (*e.g.,* modified celluloses such as microcrystalline cellulose, and cellulose ethers like hydroxypropyl cellulose (HPC) and cellulose ether hydroxypropyl methylcellulose (HPMC)), xylitol, sorbitol, mannitol, gelatin, polymers (*e.g.,* polyvinylpyrrolidone (PVP), polyethylene glycol (PEG), crosslinked polyvinylpyrrolidone (crospovidone), carboxymethyl cellulose, polyethylene-polyoxypropylene-block polymers, and crosslinked sodium carboxymethyl cellulose (croscarmellose sodium)), titanium oxide, azo dyes, silica gel, fumed silica, talc, magnesium carbonate, vegetable stearin, magnesium stearate, aluminum stearate, stearic acid, antioxidants (*e.g.,* vitamin A, vitamin E, vitamin C, retinyl palmitate, and selenium), citric acid, sodium citrate, parabens (*e.g.,* methyl paraben and propyl paraben), petrolatum, dimethyl sulfoxide, mineral oil, serum proteins (*e.g.,* human serum albumin), glycine, sorbic acid, potassium sorbate, water, salts or electrolytes (*e.g.,* saline, protamine sulfate, disodium hydrogen phosphate, potassium hydrogen phosphate, sodium chloride, and zinc salts), colloidal silica, magnesium trisilicate, polyacrylates, waxes, wool fat, lecithin, DMSO, and corn oil.

A composition containing one or more protease inhibitors and/or one or more microorganisms that produce one or more protease inhibitors can be administered to a mammal (*e.g.,* a human) in need thereof (*e.g.,* a mammal having, or at risk of developing, a gastrointestinal disorder (*e.g.,* an IBS such as PI-IBS)) by any appropriate type of administration. For example, a composition containing one or more protease inhibitors and/or one or more microorganisms that produce one or more protease inhibitors can be designed for oral administration, parenteral administration (including, without limitation, a subcutaneous, intramuscular, intravenous, intradermal, intra-cerebral, intrathecal, or intraperitoneal (i.p.) administration), and transmucosal administration (including, without limitation, a buccal, vaginal, or rectal administration) to the mammal. Compositions suitable for oral administration include, without limitation, liquids, tablets, capsules, pills, powders, gels, and granules. In some cases, compositions suitable for oral administration can be in the form of a supplement (*e.g.,* a nutritional supplement, a food supplement, or a drink supplement). For example, a supplement can be a liquid preparation formulated to contain one or more protease inhibitors and/or one or more microorganisms that produce one or more protease inhibitors. Liquid preparations for oral administration can take the form of, for example, solutions, syrups, or suspension, or they can be presented as a dry product for constitution with saline or other suitable liquid vehicle before use. For example, a supplement can be in the form of a pill, tablet, powder, liquid, or capsule formulated to contain one or more protease inhibitors and/or one or more microorganisms that produce one or more protease inhibitors. Tablets or capsules can be prepared by conventional means with pharmaceutically acceptable excipients such as binding agents, fillers, lubricants, disintegrants, or wetting agents. The tablets can be coated by methods known in the art. In some cases, compositions suitable for oral administration can be in the form of a food product formulated to contain one or more protease inhibitors and/or one or more microorganisms that produce one or more protease inhibitors. Examples of such food products include, without limitation, yogurt (*e.g.,* dairy yogurt and non-dairy yogurt), kefir, buttermilk, cheese (*e.g.,* aged cheese), milk (e.g., acidified milk), milk powder, tea, juice, candies, chocolates, chewable bars, cookies, wafers, crackers, cereals, and combinations thereof. In some cases, compositions suitable for oral administration can be formulated such that one or more protease inhibitors and/or one or more microorganisms that produce one or more protease inhibitors are encapsulated for release within the intestines of a mammal. Compositions suitable for parenteral administration include, without limitation, aqueous and non-aqueous sterile injection solutions that can contain anti-oxidants, buffers, bacteriostats, and/or solutes that render the formulation isotonic with the blood of the intended recipient.

A composition containing one or more protease inhibitors and/or one or more microorganisms that produce one or more protease inhibitors can be administered to a mammal having, or at risk of developing, a gastrointestinal disorder (*e.g.,* an IBS such as PI-IBS) in any appropriate amount (*e.g.,* any appropriate dose). Effective amounts can vary depending on the route of administration, the age and general health condition of the subject, excipient usage, the possibility of co-usage with other therapeutic treatments such as use of other agents, and the judgment of the treating physician. An effective amount of a composition containing one or more protease inhibitors and/or one or more microorganisms that produce one or more protease inhibitors can be any amount that can treat a mammal having, or at risk of developing, a gastrointestinal disorder as described herein without producing significant toxicity to the mammal. For example, an effective amount of one or more protease inhibitors can be from about 0.01 milligrams per kilogram body weight (mg/kg) to about 100 mg/kg (e.g., from about 0.01 mg/kg to about 90 mg/kg, from about 0.01 mg/kg to about 80 mg/kg, from about 0.01 mg/kg to about 70 mg/kg, from about 0.01 mg/kg to about 60 mg/kg, from about 0.01 mg/kg to about 50 mg/kg, from about 0.01 mg/kg to about 40 mg/kg, from about 0.01 mg/kg to about 30 mg/kg, from about 0.01 mg/kg to about 20 mg/kg, from about 0.01 mg/kg to about 10 mg/kg, from about 0.01 mg/kg to about 5 mg/kg, from about 0.01 mg/kg to about 1 mg/kg, from about 0.05 mg/kg to about 100 mg/kg, from about 1 mg/kg to about 100 mg/kg, from about 10 mg/kg to about 100 mg/kg, from about 20 mg/kg to about 100 mg/kg, from about 30 mg/kg to about 100 mg/kg, from about 40 mg/kg to about 100 mg/kg, from about 50 mg/kg to about 100 mg/kg, from about 60 mg/kg to about 100 mg/kg, from about 70 mg/kg to about 100 mg/kg, from about 80 mg/kg to about 100 mg/kg, from about 90 mg/kg to about 100 mg/kg, from about 1 mg/kg to about 90 mg/kg, from about 10 mg/kg to about 80 mg/kg, from about 20 mg/kg to about 70 mg/kg, from about 30 mg/kg to about 60 mg/kg, from about 40 mg/kg to about 50 mg/kg, from about 1 mg/kg to about 20 mg/kg, from about 10 mg/kg to about 30 mg/kg, from about 20 mg/kg to about 50 mg/kg, from about 30 mg/kg to about 60 mg/kg, from about 40 mg/kg to about 70 mg/kg, from about 50 mg/kg to about 80 mg/kg, or from about 60 mg/kg to about 90 mg/kg) of a mammal (*e.g.,* a human) (*e.g.,* per day). In some cases, an effective amount of one or more protease inhibitors can be about 20 mg/kg protease inhibitor(s) per day. For example, an effective amount of one or more microorganisms that produce one or more protease inhibitors can be from about 10² CFU to about 10¹⁰ CFU (e.g., from about 10² CFU to about 10⁹ CFU, from about 10² CFU to about 10⁸ CFU, from about 10² CFU to about 10⁷ CFU, from about 10² CFU to about 10⁶ CFU, from about 10² CFU to about 10⁵ CFU, from about 10² CFU to about 10⁴ CFU, from about 10² CFU to about 10³ CFU, from about 10³ CFU to about 10¹⁰ CFU, from about 10⁴ CFU to about 10¹⁰ CFU, from about 10⁵ CFU to about 10¹⁰ CFU, from about 10⁶ CFU to about 10¹⁰ CFU, from about 10⁷ CFU to about 10¹⁰ CFU, from about 10⁸ CFU to about 10¹⁰ CFU, from about 10⁹ CFU to about 10¹⁰ CFU, from about 10³ CFU to about 10⁹ CFU, from about 10⁴ CFU to about 10⁸ CFU, from about 10⁵ CFU to about 10⁷ CFU, from about 10³ CFU to about 10⁵ CFU, from about 10⁴ CFU to about 10⁶ CFU, from about 10⁵ CFU to about 10⁷ CFU, from about 10⁶ CFU to about 10⁸ CFU, or from about 10⁷ CFU to about 10⁹ CFU) (*e.g.,* per day). In some cases, an effective amount of one or more microorganisms that produce one or more protease inhibitors can be about 10⁹ CFU.

The effective amount can remain constant or can be adjusted as a sliding scale or variable dose depending on the mammal's response to treatment. Various factors can influence the actual effective amount used for a particular application. For example, the frequency of administration, duration of treatment, use of multiple treatment agents, route of administration, and/or severity of the gastrointestinal disorder in the mammal being treated may require an increase or decrease in the actual effective amount administered.

A composition containing one or more protease inhibitors and/or one or more microorganisms that produce one or more protease inhibitors can be administered to a mammal having, or at risk of developing, a gastrointestinal disorder (*e.g.,* an IBS such as PI-IBS) in any appropriate frequency. The frequency of administration can be any frequency that can treat a mammal having, or at risk of developing, a gastrointestinal disorder without producing significant toxicity to the mammal. For example, the frequency of administration can be from about once a day to about once a week, from about twice a day to about twice a week, or from about three times a day to about once a day. The frequency of administration can remain constant or can be variable during the duration of treatment. As with the effective amount, various factors can influence the actual frequency of administration used for a particular application. For example, the effective amount, duration of treatment, use of multiple treatment agents, and/or route of administration may require an increase or decrease in administration frequency.

A composition containing one or more protease inhibitors and/or one or more microorganisms that produce one or more protease inhibitors can be administered to a mammal having, or at risk of developing, a gastrointestinal disorder (*e.g.,* an IBS such as PI-IBS) for any appropriate duration. An effective duration for administering or using a composition containing one or more protease inhibitors and/or one or more microorganisms that produce one or more protease inhibitors can be any duration that can treat a mammal having, or at risk of developing, a gastrointestinal disorder without producing significant toxicity to the mammal. For example, the effective duration can vary from several weeks to several months, from several months to several years, or from several years to a lifetime. Multiple factors can influence the actual effective duration used for a particular treatment. For example, an effective duration can vary with the frequency of administration, effective amount, use of multiple treatment agents, and/or route of administration.

In some cases, methods for treating a mammal (*e.g.,* a human) having, or at risk of developing, a gastrointestinal disorder (*e.g.,* an IBS such as PI-IBS), can include administering to the mammal one or more protease inhibitors and/or one or more microorganisms that produce one or more protease inhibitors as the sole active ingredient to treat the mammal. For example, a composition containing one or more protease inhibitors and/or one or more microorganisms that produce one or more protease inhibitors can include the one or more protease inhibitors and/or one or more microorganisms that produce one or more protease inhibitors as the sole active ingredient in the composition that is effective to treat a mammal having, or at risk of developing, a gastrointestinal disorder.

In some cases, methods for treating a mammal (*e.g.,* a human) having, or at risk of developing, a gastrointestinal disorder (*e.g.,* an IBS such as PI-IBS) as described herein (*e.g.,* by administering one or more protease inhibitors and/or one or more microorganisms that produce one or more protease inhibitors) also can include administering to the mammal one or more (*e.g.,* one, two, three, four, five or more) additional active agents (*e.g.,* therapeutic agents) that are effective to treat a gastrointestinal disorder (*e.g.,* that are effective to treat one or more symptoms of a gastrointestinal disorder) to treat the mammal. Examples of additional active agents that can be used as described herein to treat a gastrointestinal disorder include, without limitation, alosetron (LOTRONEX^{®}), eluxadoline (VIBERZI^{®}), rifaximin (XIFAXAN^{®}), lubiprostone (AMITIZA^{®}), linaclotide (LINZESS^{®}), plecanatide (e.g., TRULANCE^{®}), prucalopride (e.g., Prudac^{™}), ramosetron, tegaserod, tenapanor (e.g., IBSRELA^{®}), probiotics (*e.g., Bifidobacterium, Lactobacillus,* and *Saccharomyces boulardii),* prebiotics, peppermint, fiber supplements (*e.g.,* psyllium (METAMUCIL^{®}), laxatives (*e.g.,* magnesium hydroxide (Phillips'^{®} Milk of Magnesia), and polyethylene glycol (MiraLAX^{®}), anti-diarrheal medications (*e.g.,* loperamide (IMODIUM^{®}), bile acid binders *(e.g.,* cholestyramine (PREVALITE^{®}), colestipol (COLESTID^{®}), and colesevelam (WELCHOL^{®})), anticholinergic medications (*e.g*., dicyclomine (BENTYL^{®}), tricyclic antidepressants (*e.g.,* a lower than normal dose of a tricyclic antidepressant such as imipramine (TOFRANIL^{™}), desipramine (NORPRAMINE^{®}), and nortriptyline (PAMELOR^{™})), selective serotonin reuptake inhibitor (SSRI; *e.g.,* fluoxetine (PROZAC^{®} and SARAFEM^{®}), and paroxetine (PAXIL^{®})), and pain medications (*e.g.,* pregabalin (LYRICA^{®}) and gabapentin (NEURONTIN^{®})). In some cases, the one or more additional active agents can be administered together with the administration of the one or more protease inhibitors and/or one or more microorganisms that produce one or more protease inhibitors. For example, a composition containing one or more protease inhibitors and/or one or more microorganisms that produce one or more protease inhibitors also can include one or more additional active agents that are effective to treat a gastrointestinal disorder. In some cases, the one or more additional active agents that are effective to treat a gastrointestinal disorder can be administered independent of the administration of the one or more protease inhibitors and/or one or more microorganisms that produce one or more protease inhibitors. When the one or more additional active agents that are effective to treat a gastrointestinal disorder are administered independent of the administration of the one or more protease inhibitors and/or one or more microorganisms that produce one or more protease inhibitors, the one or more protease inhibitors and/or one or more microorganisms that produce one or more protease inhibitors can be administered first, and the one or more additional active agents that are effective to treat a gastrointestinal disorder administered second, or vice versa.

In some cases, methods for treating a mammal (*e.g.,* a human) having, or at risk of developing, a gastrointestinal disorder (*e.g.,* an IBS such as PI-IBS) as described herein (*e.g.,* by administering one or more protease inhibitors and/or one or more microorganisms that produce one or more protease inhibitors) also can include subjecting the mammal to one or more (*e.g.,* one, two, three, four, five or more) additional treatments (*e.g.,* therapeutic interventions) that are effective to treat a gastrointestinal disorder (*e.g.,* that are effective to treat one or more symptoms of a gastrointestinal disorder) to treat the mammal. Examples of additional treatments that can be used as described herein to treat a gastrointestinal disorder include, without limitation, changes in diet such as eating high-fiber foods, drinking plenty of fluids, avoiding high-gas foods (*e.g.,* raw fruit, certain vegetables such as cabbage, broccoli and cauliflower), avoiding high-gas beverages (*e.g.,* carbonated beverages, alcoholic beverages, caffeinated beverages), avoiding gluten, avoiding fermentable oligo-, di-, and monosaccharides and polyols (FODMAPs); exercising regularly; getting enough sleep; hypnosis; mindfulness training; acupuncture; and stress reduction. In some cases, the one or more additional treatments that are effective to treat a gastrointestinal disorder can be performed at the same time as the administration of the one or more protease inhibitors and/or one or more microorganisms that produce one or more protease inhibitors. In some cases, the one or more additional treatments that are effective to treat a gastrointestinal disorder can be performed before and/or after the administration of the one or more protease inhibitors and/or one or more microorganisms that produce one or more protease inhibitors.

In some cases, methods for treating a mammal (*e.g.,* a human) having, or at risk of developing, a gastrointestinal disorder (*e.g.,* an IBS such as PI-IBS) as described herein (*e.g.,* by administering one or more protease inhibitors and/or one or more microorganisms that produce one or more protease inhibitors) also can include monitoring the mammal being treated. For example, methods described herein also can include monitoring the severity or progression of a gastrointestinal disorder in a mammal. Any appropriate method can be used to monitor the severity or progression of a gastrointestinal disorder in a mammal. In some cases, one or more symptoms of a gastrointestinal disorder can be assessed using any appropriate methods and/or techniques, and can be assessed at different time points. For example, physical examination, Rome criteria, Manning criteria, imaging tests (*e.g.,* flexible sigmoidoscopy, colonoscopy, upper endoscopy, X-ray, and computerized tomography (CT) scan), laboratory tests (*e.g.,* lactose intolerance tests, breath test for bacterial overgrowth, and stool tests), gastrointestinal transit, anorectal barostat, small bowel or fecal microbiome assessments, and/or gastrointestinal permeability can be used to assess a gastrointestinal disorder. In some cases, methods described herein can include monitoring a mammal being treated as described herein for toxicity. The level of toxicity, if any, can be determined by assessing a mammal's clinical signs and symptoms before and after administering a known amount of a particular composition. It is noted that the effective amount of a particular composition administered to a mammal can be adjusted according to a desired outcome as well as the mammal's response and level of toxicity.

The invention will be further described in the following examples, which do not limit the scope of the invention described in the claims.

### EXAMPLES

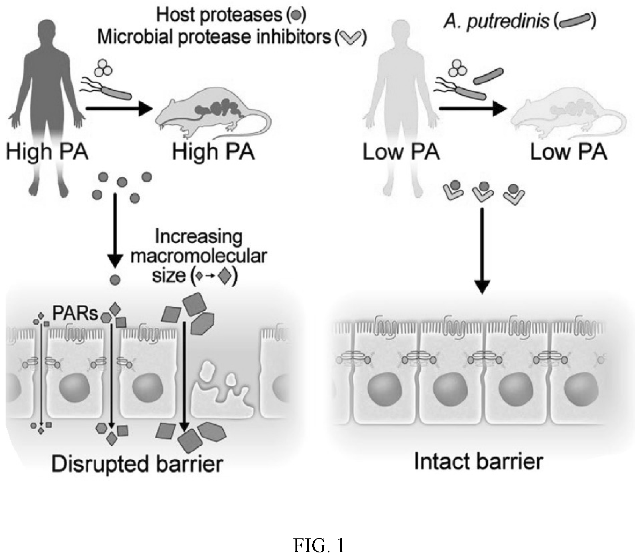

Without being bound by theory, it is believed that, in PI-IBS patients, host serine proteases regulated in a microbiota dependent manner disrupt barrier function (Figure 1). These Examples demonstrate that alterations in commensal gut microbiota drives proteolytic activity in PI-IBS and that host serine proteases disrupt barrier function in PI-IBS.

### Example 1: High PA in PI-IBS results from impaired microbial inhibition of host serine proteases

### PI-IBS patients have high fecal PA

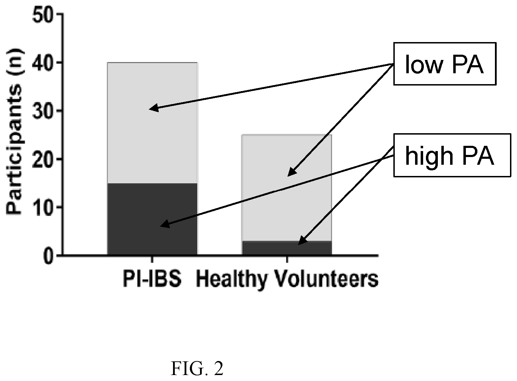

Fecal supernatants were prepared by diluting in PBS, followed by homogenization, centrifugation and filtration using 0.2 µm filters. Using a casein substrate-based assay for proteolytic digestion, 15/40 (37%) PI-IBS patients demonstrated high PA in fecal supernatants (defined as >85 percentile of the healthy group, 891 Nα-Benzoyl-L-arginine ethyl ester (BAEE) /mg of protein) as compared to 3/25 (12%) healthy volunteers (Figure 2).

### Human fecal metaproteomics reveals human serine proteases in greater abundance in high PA PI-IBS patients

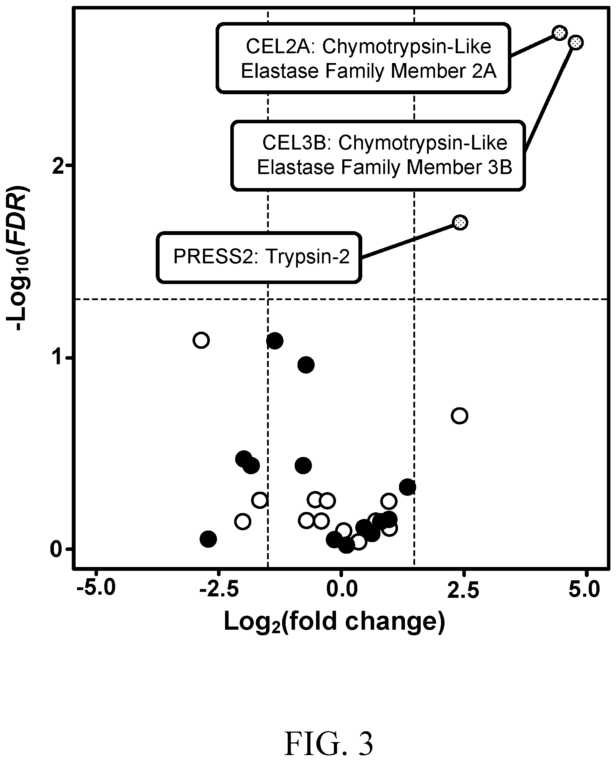

Hundreds of host and bacterial proteases exist in the intestinal tract. The nature and origin of proteases driving differences in fecal PA among high and low PA patients was determined. Prior targeted approaches can only identify a minor subset of those proteases and cannot distinguish between host and microbial origin. Thus, fecal metaproteomics pipeline was developed. Briefly, protein was extracted from 7 high PA and 6 low PA fecal supernatants and SDS gel electrophoresis was performed. Four protein bands were extracted (0-20, 20-37, 37-70, >70 KDa) and mass spectrometry (MS/MS) was performed. Microbial shotgun metagenomic data were used to generate a microbial metapeptide database which was combined with human UniProt based peptide database. The MS/MS output from fecal proteins was searched against this database using MaxQuant. MEROPS, a large publicly available database of proteases and protease inhibitors was also used to match with the output. Quantitative data was cyclic Loess normalized using Limma software and analyzed using probe-level expression change averaging to obtain differentially abundant proteins. High and low PA fecal supernatants had 1210 (588 human) and 2801 (755 human) unique peptides respectively. Proteases in general constituted 12% of fecal proteins. Chymotrypsin like pancreatic elastase 2A and 3B were the 2-topmost differentially expressed proteins (multiple correction *FDR*<0.01, Standard t-test), both demonstrating ~4.5-fold greater log2fold abundance in high PA supernatants. Trypsin-2 was also 2.5 log2fold more abundant in high PA supernatants (*FDR*<0.05, Figure 3).

### Colonic mucosal protease expression and activity was similar between high and low PA patients

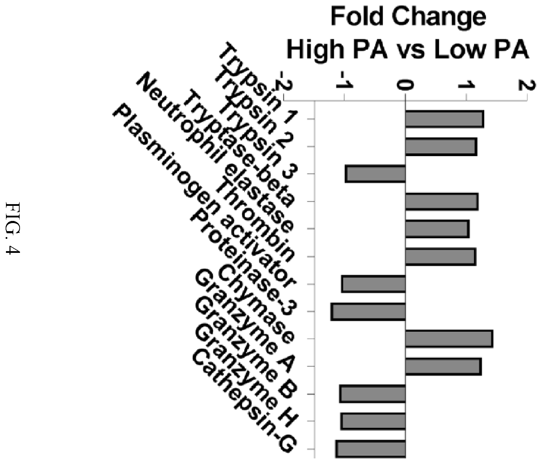

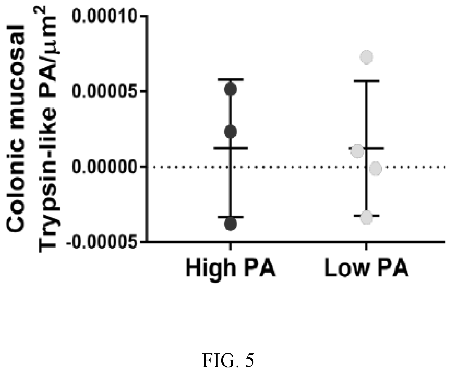

To determine if colonic mucosal protein expression of proteases differ between high and low PA patients, proteomics were performed on sigmoid colonic mucosal biopsies on a preliminary set of high and low PA patients using the SOMAscan protein assay that quantitatively identifies 1305 human proteins. Although several serine proteases were identified, there was no difference in abundance between high and low PA patients (Figure 4). Additionally, *in situ* zymography was performed using sigmoid colonic biopsies from a pilot set of high and low PA patients incubated overnight with substrate N-p-Tosyl-Gly-Pro-Arg 7-amido-4-methylcoumarin HCl (AMC), imaged and florescence quantified. No evidence was found for increased tissue PA (Figure 5).

### High and low PA subjects have differences in microbiota composition

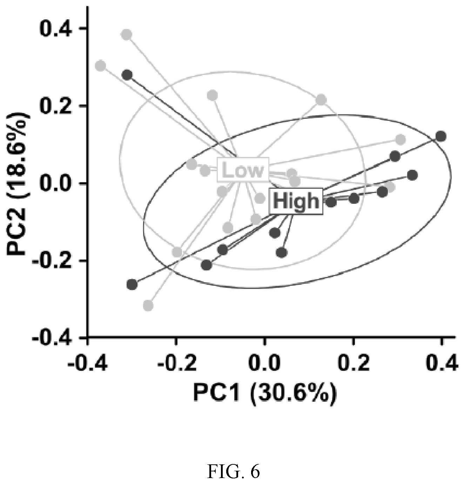

Fecal shotgun metagenomics performed on a pilot set of high PA patients and healthy volunteers (predominantly low PA) demonstrated differences in beta diversity suggesting differences in microbial composition (Bray-Curtis, *p*=0.02 PERMANOVA) after adjusting for age, sex and BMI (Figure 6).

### Mice humanized with high PA PI-IBS microbiota show PA similar to GF state

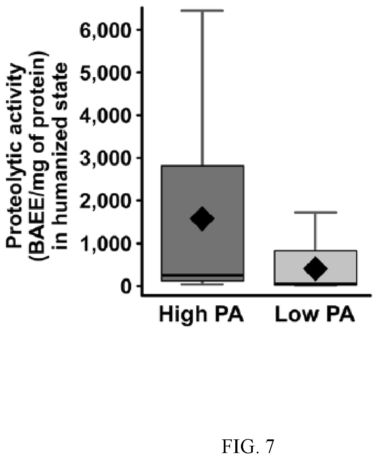

In order to "standardize" the host and to specifically study the microbiota-driven regulation of PA, a humanized mouse model was used. GF mice had 10-fold higher fecal PA than conventional mice (1754 vs 171 BAEE/mg of protein, *p*<0.05). A single oral gavage of commensal microbial community from the high and low PA patients to 4-week old GF Swiss-Webster female mice was performed. Fecal PA was assessed in GF (pre-gavage) state and 6 weeks following the gavage to allow establishment of the microbial community (i.e., humanization). Mice humanized with high PA PI-IBS human stool had 3.7-fold higher fecal PA at 6 weeks as compared to mice humanized with healthy volunteer (low PA) stool (1570 vs 419 BAEE/mg protein, *p*=0.07) (Figure 7). Low PA humanized mice only had 26% PA of the GF state left compared to 100% in high PA humanized mice.

### Mouse with high PA after humanization has higher serine proteases than mouse with low PA after humanization

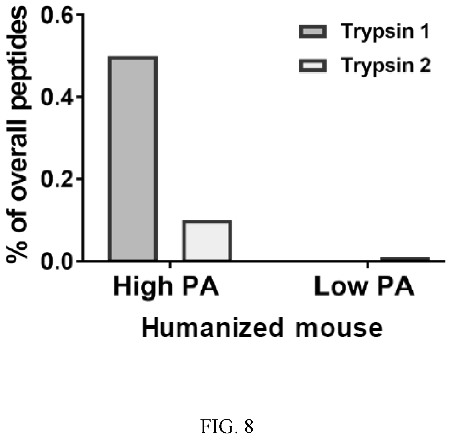

In order to determine changes in the luminal protease environment upon humanization with unique microbial communities, metaproteomics were performed on a pilot set of humanized mice fecal samples. Fecal protein was subjected to MS/MS and MetaQuant quantified peptides were linked to mouse protein database. Mouse with high PA after humanization showed greater abundance of peptides that could be mapped to proteases in MEROPS as compared to the mouse with low fecal PA (27% vs 17%). Additionally, trypsin-1 and trypsin-2 abundance was higher in the high PA mouse as compared to the low PA mouse (Figure 8).

### PA changes dramatically in the cecum

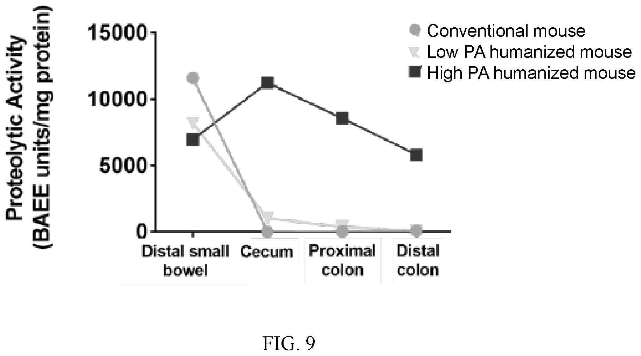

Regional assessment of PA in luminal contents from distal small bowel, cecum, proximal colon and distal colon showed that PA decreases in the cecum in the mouse humanized with low PA microbiota (Figure 9). Conventional mouse also showed a similar PA drop in cecum. However, the mouse humanized with high PA microbiota show uninhibited PA in cecum followed by some decline upon colonic transit.

### Changes in protease and protease inhibitors upon humanization with high and low PA microbiota

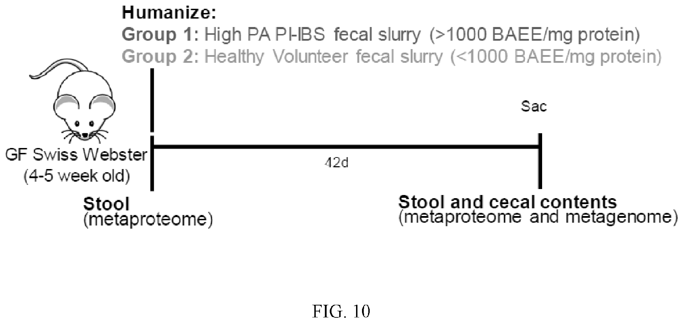

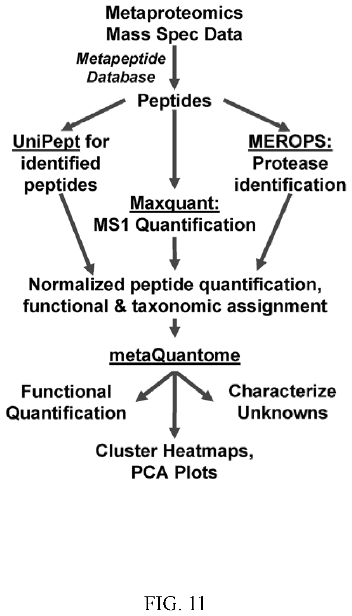

Humanized mice are used as a platform for studying the effects of commensal microbiota from high PA PI-IBS patients (>1000 BAEE/mg of protein) and low PA healthy volunteers (<1000), on proteases and protease inhibitors. 40 unique humanized mice states are created: 20 high PA and 20 healthy (10/sex/group). Studies are described in Figure 10. Fecal metaproteomics are performed on longitudinal samples collected from the same mouse in GF state and humanized state. Longitudinal shifts in protease and protease inhibitors are compared. Additionally, differences in fecal and cecal metaproteome in humanized state are compared between the two groups. To allow comparison of metaproteome in humanized state, a microbial metapeptide library is created. For this, microbial shotgun metagenomics are performed on fecal and cecal samples collected in humanized state. The metaproteomics flow is optimized as described above. Briefly, protein is extracted from fecal supernatants using acetone precipitation to remove impurities/non-proteins, reconstituted and run on SDS-PAGE. Gels are stained with Coomassie blue and imaged. 0-20, 20-37 and 37-70 KDa bands are excised to study most bacterial protease inhibitors, host and/or bacterial proteases and host protease inhibitors, respectively. MS/MS analysis is done using the SCIEX 5600 TripleTOF instrument. Using a Galaxy workflow (Figure 11), MS/MS data is searched against a metapeptide database (generated using Sixgill61 and guided by metagenomic data from the same samples) using search algorithms. Identified peptides are assigned quantification values (using MaxQuant), taxonomy and function (using UniPept) and normalized to generate a tabular output. Data from MEROPS are added to the output. The metaQuantome tool within Galaxy is used to generate quantitative information for functional analysis and to quantitate expression of unassigned peptides.

### Protease inhibitor activity in humanized mice

To determine the activity of protease inhibitors in the same cohort of high and low PA humanized mice, the inhibitory potential of luminal protease inhibitors is studied. Whether the low PA cecal and fecal supernatants can cause inhibition of exogenous trypsin and high PA fecal supernatant activity *in vitro* is determined using inhibitor zymography. Briefly, low PA supernatants are run through a sepharose column to remove available serine proteases, and the flow through is obtained which contains putative protease inhibitors. High PA supernatant is run on two casein-zymogram gels, one conventional and one containing flow through from low PA supernatant. Protein is then renatured using renaturation buffer and incubated overnight at 37°C. Reduction in cleared substrate between the conventional and low PA containing gels indicates protease inhibition by low PA supernatants. Further, bands inhibited are excised for characterization of peptides using MS/MS.

### Example 2: High PA PI-IBS is due to absence of Alistipes putredinis

### High PA PI-IBS patients have an absence of A. putredinis

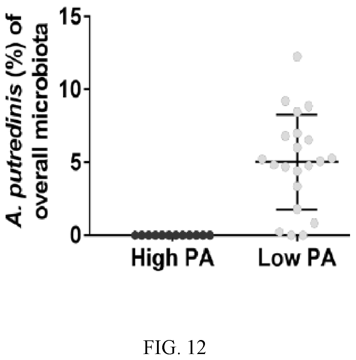

Species-level taxonomy revealed the most statistically significant difference in abundance of *A. putredinis* (*q*=0.03) between the high and low PA groups. Seven other species showed differential abundance at *q*<0.05 threshold, however they all had abundances <1% in both the groups. None of the high PA PI-IBS patients possessed this commensal, whereas, the low PA patients had an average prevalence of 5% (Figure 12).

### Alistipes putredinis produced 187 protease inhibitor family is absent in high PA PI-IBS subjects and its abundance negatively correlates with overall PA

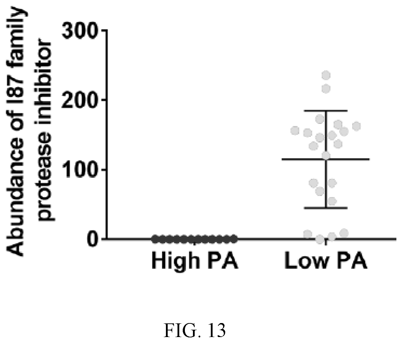

A multivariate association analysis was performed using the package MaAsLin2 to determine which MEROPS protease inhibitor families (and their putative carriers) are differentially abundant between the high and low PA groups. It was noted that among the top protease inhibitor families that were found to be significantly different, most of them were carried by bacteria from the genus *Alistipes* (385/2502). Specifically, within the MEROPS I87 protease inhibitor family, the I87 inhibitor from *A. putredinis* was found to be most significantly associated with the lower overall PA of the cohort (*FDR*=0.001). All of the high PA PI-IBS fecal samples lacked this family of inhibitors (Figure 13).

### Fecal microbial transplantation with A. putredinis enriched low PA community restores fecal PA of the high PA humanized mice

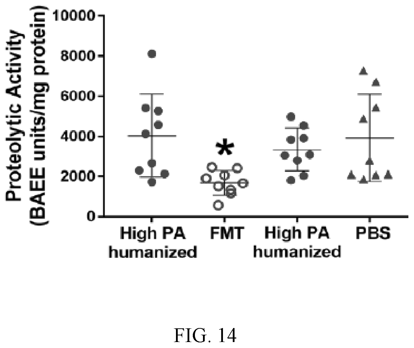

High PA humanized mice were gavaged with fecal microbiota from a healthy (low PA) patient or PBS (vehicle). Four days post gavage, mice administered low PA human feces had reduction in fecal PA, whereas, the vehicle gavaged mice retained high PA similar to the baseline (Figure 14). The microbial community associated with low PA state partially reversed the effects of high PA PI-IBS community. The donor (low PA) community had 8.8% of *A. putredinis* compared to the recipient (high PA) community which lacked that commensal.

### Microbiota associated with protease inhibition in PI-IBS patients

Shotgun metagenomics are performed on fecal samples from 66 high PA PI-IBS patients (screened from 180 PI-IBS patients, 37%), and 60 healthy volunteers. Associations between microbial diversity, composition and PA are made after controlling for age, sex and BMI. Additionally, taxonomic differences between the high and low PA groups are determined. An average sequencing depth of ~5 million reads/sample are targeted in a hope to provide species level resolution. Furthermore, metabolic pathways between the high and low PA fecal samples are determined to identify possible differences in gene content associated with enzymatic and metabolic microbial machinery between the groups.

### A. putredinis to suppress PA in humanized mice

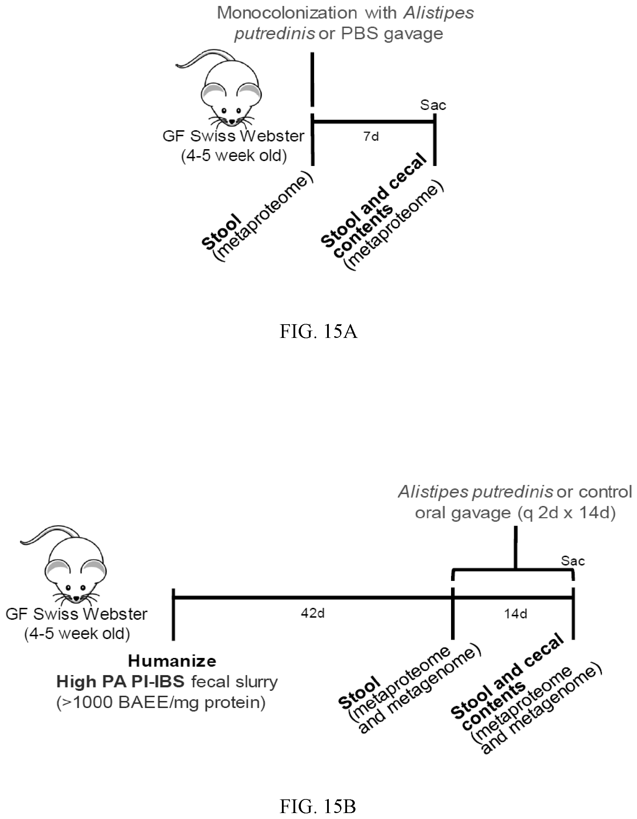

To determine specific effects of *A. putredinis* on the protease milieu, 4-week-old GF Swiss-Webster mice are monocolonized with *A. putredinis* using a single oral gavage of 10⁹ CFU suspended in PBS or PBS alone as the control (Figure 15A). Monocolonization is confirmed by stool cultures. Baseline and one-week post gavage fecal samples are collected for metaproteome analysis in the monocolonized or control gavaged mice. Cecal samples are also collected at 1 week for comparison between treated and control groups. This experiment will help determine specific effects of this species on host proteases. The metaproteome analysis will help determine the specific proteases inhibited and allow identification of the protease inhibitors produced. Ten mice (5/sex) will be used for gavage with *A. putredinis* or placebo (5/group).

To further determine specific effects of *A. putredinis* on the protease milieu, oral *A. putredinis* (or control) is administered to high PA PI-IBS humanized mice every other day for 2 weeks and changes in fecal PA, specific proteases and protease inhibitors are determined using metaproteomics (Figure 15B). Cecal samples are also collected after the 2 weeks for comparison between treated and control groups. Humanized mice are developed using 6 (3/sex) different high-PA PI-IBS associated microbiota. Ten mice are humanized with each human stool to allow 5 for *A. putredinis* and 5 for control treatment.

### Example 3: Serine proteases increase paracellular leak pathway permeability in vitro through cleavage of PARs

### Fecal supernatants from high PA patients disrupt intestinal barrier in vitro

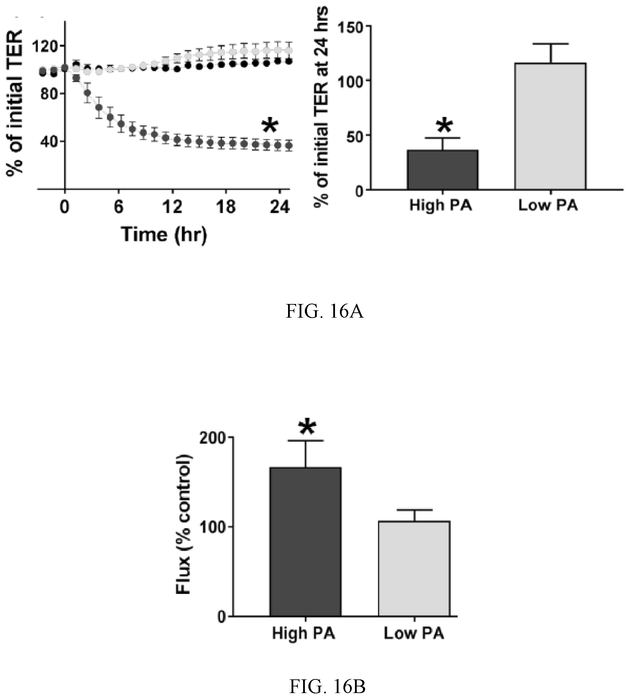

Apical addition of high PA fecal supernatants on Caco-2 monolayers caused a drop in transepithelial resistance (TER) which was not seen with low PA supernatant (Figure 16A). Additionally, high PA but not low PA fecal supernatant caused Texas red dextran (MW 3000 g/mol, radius 13 Å) flux suggesting increased permeation through the leak pathway (Figure 16B). Since pancreatic elastases were found as the most abundant proteases driving the fecal PA, their effects on barrier were tested. It was demonstrated that porcine pancreatic elastase (1 mM) increases Texas red dextran flux across Caco-2 monolayers (24 hour basolateral concentration, 5.9 vs 0.1 µg/mL for control, n=4/group, Mann Whitney, *p*<0.05). Trypsin, the other protease increased in high PA samples had an effect on barrier.

### Serine protease inhibitors block the effect of fecal supernatants on barrier

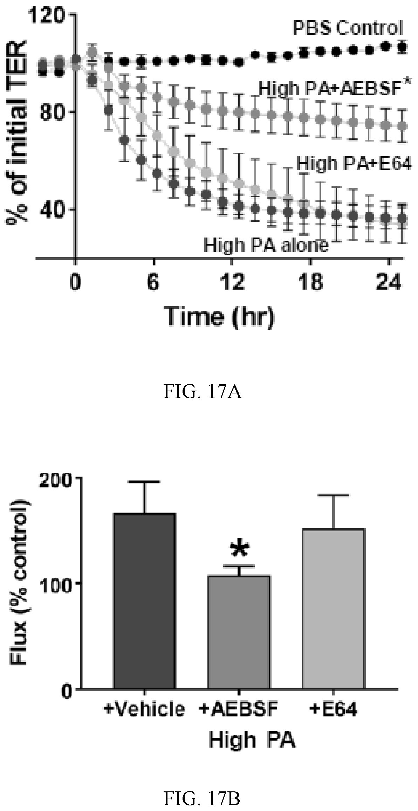

Apical preincubation with broad serine protease inhibitor (0.2 mM AEBSF) or cysteine protease inhibitor (0.1 mM E64), caused inhibition of PA (67-68%, *p*<0.05 for both). However, fecal supernatant induced TER drop was significantly inhibited by AEBSF but not by E64 (Figure 17A). Increased paracellular flux caused by high PA supernatants was also significantly reduced by AEBSF but not by E64 (Figure 17B).

### Barrier dysfunction induced by high PA fecal supernatant is partially mediated by PAR-2 receptors

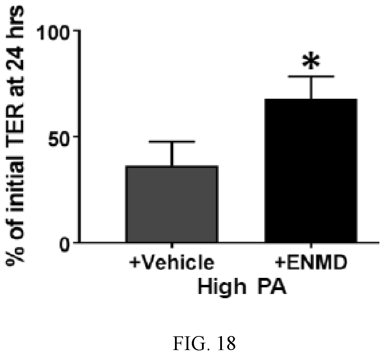

Preincubation of Caco-2 monolayer with ENMD1068 (150 µM), a selective PAR-2 antagonist, resulted in 45% inhibition of TER drop induced by high PA fecal supernatants (Figure 18).

### Fecal supernatants from high PA patients alter tight junction proteins

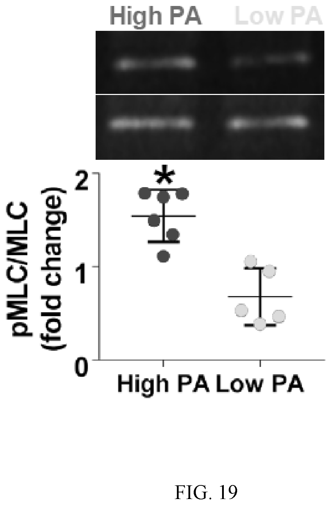

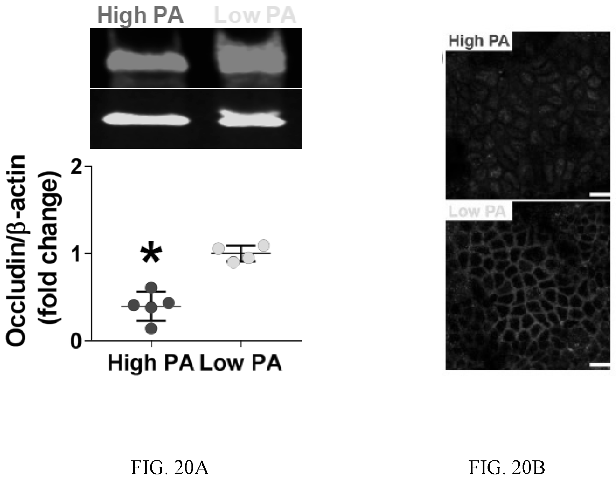

Apical incubation of Caco-2 monolayers with high PA fecal supernatants (6 hour) resulted in phosphorylation of myosin light chain (MLC) (Figure 19). Additionally, a decrease in total occludin expression was seen (Figure 20).

### Determine role of PARs in PI-IBS fecal supernatant induced barrier disruption

Caco-2 monolayers are preincubated with PAR-2 inhibitor (ENMD 1068 150 µM) alone or in combination with PAR-1 (SCH 79797 dihydrochloride 70 nM) and/or PAR-4 (ML 354 140 nM) inhibitors on apical side and determine barrier effects of two different ranges of high PA fecal supernatants (1000-3000, >3000 BAEE/mg of protein). PAR-1, 2 and 4 agonists and control peptides are used as positive and negative controls.

To compliment pharmacological inhibition, PAR-2 siRNA is used alone and in combination with PAR-1 and PAR-4 siRNA on Caco-2 monolayers. For barrier assessment, (a) continuous TER monitoring using cellZscope over 24 hours; (b) flux of 13C mannitol (MW 188 g/mol, radius 3.2 Å; pore pathway), Texas red dextran (3000 g/mol, radius 13 Å, leak pathway) and Cy5 dextran (70, 000 g/mol, unrestricted pathway) at various time-points following exposure to fecal supernatants (2, 6, and 24 hours); and (c) charge selectivity using NaCl dilution potentials are used. Size selectivity using bi-ionic potentials is measured by replacing basal Na+ (radius, 0.95 Å) with larger cations, methylamine (1.9 Å), ethylamine (2.3 Å), tetramethylammonium (2.8 Å), tetraethylammonium (3.3 Å), and N-methyl-d-glucamine (3.7 Å). This is done at 6 hours following fecal supernatant exposure.

To determine expression of tight junction genes involved in PAR mediated barrier dysfunction, commercially available NanoString microarrays are customized for the genes of interest and assess changes at 2 time-points (6 and 24 hours) following exposure of two ranges of fecal supernatant PA on Caco-2 monolayers. Additionally, levels of key tight junction proteins are determined using western blotting, and immunofluorescence (occludin, ZO-1, claudins 1, 2, E-cadherin). Western blot is done in both detergent soluble and insoluble fractions to determine membrane vs cytoplasmic distribution of proteins. The involvement of MLC phosphorylation is determined by measuring MLCK and pMLC/MLC protein levels. ML-9, a Ca²⁺ calmodulin dependent inhibitor of MLCK is used to study possible inhibition of fecal supernatant effects on barrier. Additionally, colonoids from long MLCK-/- mice are developed to determine the MLCK dependent effects of fecal supernatants.

### Activity of specific serine proteases

Activity-based probes for the five abundant classes of serine proteases are used to determine the biologically active proteases in the fecal supernatants. These are based on covalent binding of active proteases to the reactive group on the probe that mimics enzymatic substrate. Trypsin-like, chymotrypsin-like, neutrophil elastase, pancreatic elastase and kallikrein proteolytic activities are determined using their respective substrates N-p-Tosyl-Gly-Pro-Arg-AMC, Suc-Ala-Ala-Pro-Phe-AMC, Suc-Ala-Ala-Pro-Val-AMC, Suc-Ala-Ala-Ala-AMC and Pro-Phe-Arg-AMC. Each human fecal supernatant is tested in triplicate and aggregates compared between the three groups.

### Example 4: High PA PI-IBS patients have increased paracellular leak pathway permeability

### High PA PI-IBS patients have worse diarrhea

High PA PI-IBS patients have looser bowel movements (average Bristol stool scale 5.1 for high vs 4.3 for low PA, n=15 high PA and 25 low PA, *p*=0.01 Mann-Whitney) and more bowel movements per day (average 2.4 for high vs 1.8 for low PA, *p*=0.07). Within high PA, those with PA>3000 had worse diarrhea compared to PA 1000-3000 (2.8 vs 1.9 bowel movements/day, *p*=0.05).

### In vivo colonic barrier is impaired in high PA PI-IBS patients

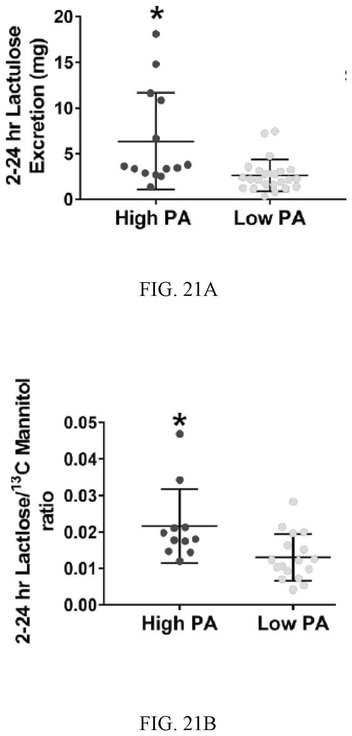

To determine barrier function in high PA PI-IBS patients, *in vivo* permeability measurement was done using a 24 hour saccharide excretion assay. As compared to low PA volunteers, high PA PI-IBS patients demonstrated greater 2-24 hour lactulose (MW 342 g/mol; radius 4.5 Å) excretion as well as greater lactulose/13C mannitol excretion ratio suggestive of increased *in vivo* colonic permeability (Figure 21). Together, this suggests greater colonic permeability through the leak paracellular pathway (radius 4-50 Å).

### Mice humanized with high PA PI-IBS microbiota have higher in vivo permeability as compared to those humanized with low PA microbiota

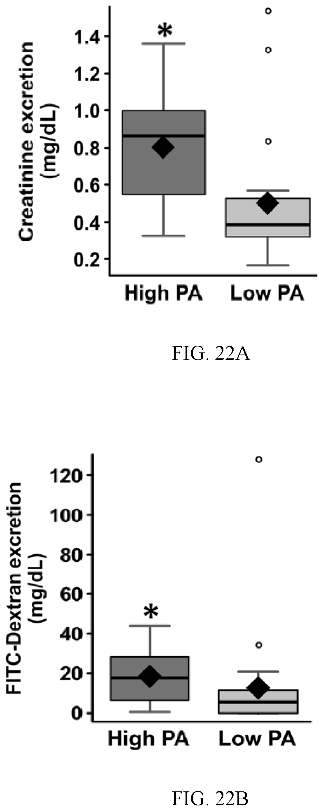

*In vivo* permeability of the humanized mice was assessed at 6 weeks. Three tracers (Creatinine, 400 Da; FITC Dextran, 4 kDa; RITC Dextran, 70 kDa) were orally gavaged, and serum was obtained 5 hours post-gavage using intracardiac puncture. Mice humanized with high PA stool had greater *in vivo* permeability for creatinine (0.81 vs 0.50 mg/dL, p<0.05) and FITC Dextran (19.1 vs 13.68 mg/dL, *p*<0.05) as compared to low PA humanized mice (Figure 22). The permeability of RITC Dextran did not differ between the two groups. Additionally, GF mice exhibited greater permeability for all three tracers as compared to any of the humanized states. This suggests that regulation of PA by commensal microbes plays a role modulating *in vivo* intestinal permeability.

### Mucosal barrier pathways affected in high PA PI-IBS patients

To determine flux of 13C mannitol (MW 188 g/mol), Texas red dextran (3000 g/mol) and Cy5 dextran (70, 000 g/mol) across colonic mucosal biopsies, biopsies from the sigmoid colon (25-30 cm from anal verge) are obtained during a flexible sigmoidoscopy. Three biopsies from each patient are mounted in Ussing chambers. Transmucosal resistance at baseline and apical to basolateral flux (every 30 minutes for 3 hours) are quantified and compared between 2 groups of high PA PI-IBS patients and healthy volunteers. It is noted that a limitation of biopsies is the short viability (~4 hours) in Ussing chambers. Recently, organoids have emerged as a useful tool for assessment of barrier function providing complex architecture and cell types more closely resembling human epithelium. These can be especially relevant for human studies where *in vivo* testing is often difficult.

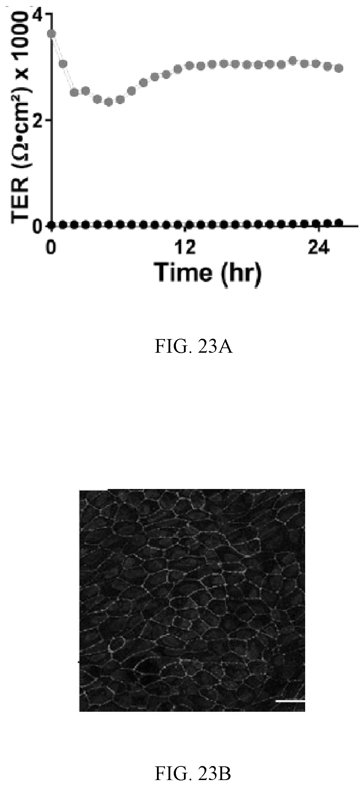

Colonoids from high and low PA patients are developed and differences in the TER and macromolecular flux across them are determined. These findings are compared among the three groups and also correlated with the biopsy findings from the same patients. To demonstrate feasibility, human crypt derived colonoid monolayers were developed to study barrier as demonstrated by continuous TER tracing on cellZscope (Figure 23A) and immunofluorescence (Figure 23B).

Colonic biopsies are used for quantification of tight junction proteins using western blotting (detergent soluble and insoluble fractions), and immunofluorescence (occludin, ZO-1, claudins 1, 2, E-cadherin). pMLC and MLCK expression levels are determined.

### Barrier pathways affected in high PA humanized mice

It was determined that high PA humanized mice have increased *in vivo* paracellular permeability for molecules permeable by both pore and leak pathways. To determine PAR-2 and MLCK dependent effects of intraluminal proteases, PAR-2-/- mice derived GF and derive MLCK-/- mice GF are obtained. These are humanized with high and low PA associated microbiota (3/sex). *In vivo* permeability is done using the three tracers (Creatinine, 400 Da; FITC Dextran, 4 kDa; RITC Dextran, 70 kDa) as described above.

To determine if luminal inhibition of PA can restore/improve the *in vivo* permeability seen in the high PA state, nafamostat (oral gavage, 20 mg/kg body weight), an established protease inhibitor, is used daily over the course of 7 days. Control mice receive an equivalent gavage volume of PBS. Considering the protease inhibitory potential of *A. putredinis, A. putredinis* or PBS gavaged high PA mice are used. Fecal samples are collected at baseline and at the end of treatment. *In vivo* permeability is tested in the treated and control mice. A total of 6 (3/sex) high PA humanized states are tested. 16 mice are humanized to allow 4/group for *in vivo* permeability assessment (3 gavaged with dyes and 1 with PBS for background).

### Example 5: Bacterial GUS activity and inhibition of serine proteases

To determine if mono association with GUS overexpressing *E. coli* results in inhibition of fecal PA, plasmid pOPS0750, with constitutive expression of bacterial GUS A, (Addgene Plasmid # 115511), were used to transform ArcticExpress (DE3) Competent *E*. *coli* (Agilent), and successful transformants were selected using Luria Bertani plates with Kanamycin and Tetracycline. Fecal pellets were collected from 10 week old GF mice, followed by gavage of 200 µL of either GUS⁺ or control non-transformed DE3 *E. coli,* under aseptic conditions. After 7 days, GF mice gavaged with GUS⁺ *E. coli* had significantly lower trypsin-like PA than those gavaged with control *E. coli* (Figure 10). These results demonstrate that microbial GUS overexpression *in vivo* results in an effective inhibition of PA, highlighting a novel role for microbiota in regulating luminal PA.

To determine if members of the *Alistipes* genus are associated with bilirubin deconjugation, a correlation between abundance of *Alistipes* genus and other candidate microbes with fecal urobilin levels was plotted. A strong, statistically significant correlation was seen for *A. putredinis* as well as other members of *Alistipes* genus. A correlation was also found for *B. ovatus* and *P. copri.* More so, compared to healthy controls, IBD patients had lower abundance of these taxa. These results demonstrate that microbes lost in high PA dysbiosis can produce GUS, which is involved in bilirubin deconjugation.

### Example 6: Treating IBS

A human identified as having IBS (*e.g.,* PI-IBS) is administered a composition (e.g., a composition formulated for oral administration) including one or more microorganisms containing nucleic acid encoding a glucuronidase polypeptide (e.g., a beta-glucuronidase polypeptide). In some cases, a microorganism can include exogenous nucleic acid encoding a glucuronidase polypeptide (e.g., a beta-glucuronidase polypeptide) that is driven by a strong promoter. For example, one or more microorganisms can be engineered to include and express exogenous nucleic acid encoding a beta-glucuronidase polypeptide such that the expressed beta-glucuronidase polypeptide converts conjugated bilirubin to unconjugated bilirubin within the mammal's gastrointestinal tract.

The administered composition (e.g., a composition formulated for oral administration) including one or more microorganisms containing nucleic acid encoding a glucuronidase polypeptide (e.g., a beta-glucuronidase polypeptide), can be effective to reduce the severity of one or more symptoms of IBS (*e.g.,* PI-IBS).

### Example 7: Exemplary Embodiments

Embodiment 1. A nutritional supplement comprising one or more microorganisms selected from the group consisting of *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.*
Embodiment 2. The nutritional supplement of embodiment 1, wherein said microorganism is encapsulated to be released in the intestine of a mammal.
Embodiment 3. The nutritional supplement of embodiment 1 or embodiment 2, wherein said nutritional supplement is selected from the group consisting of a liquid, a tablet, a capsule, a pill, a powder, a gel, and granules.
Embodiment 4. The nutritional supplement of any one of embodiments 1-3, wherein said nutritional supplement comprises a protease inhibitor.
Embodiment 5. The nutritional supplement of embodiment 4, wherein said protease inhibitor is selected from the group consisting of 4-benzenesulfonyl fluoride hydrochloride (AEBSF), nafamostat, secretory leucocyte protease inhibitor (SLPI), serpins, elafin, a host metabolite, and a bacterial metabolite.
Embodiment 6. The nutritional supplement of embodiment 4, wherein said protease inhibitor inhibits the activity of a protease selected from the group consisting of trypsin-1, trypsin-2, trypsin-3, chymotrypsin like elastase 2A, chymotrypsin like elastase 3B, chymotrypsin, a kallikrein, plasmin, and thrombin.
Embodiment 7. A food product comprising a microorganism comprising nucleic acid encoding a protease inhibitor, wherein said microorganism is selected from the group consisting of *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.*
Embodiment 8. The food product of embodiment 7, wherein said product contains between 10² colony forming units (CFU) to about 10¹⁰ CFU of said microorganism.
Embodiment 9. The food product of embodiment 7 or embodiment 8, wherein said food product is selected from the group consisting of yogurt, kefir, buttermilk, cheese, milk, milk powder, tea, juice, cookies, wafers, crackers, and cereals.
Embodiment 10. The food product of any one of embodiments 7-9, said food product comprising *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.*
Embodiment 11. The food product of any one of embodiments 7-10, wherein said protease inhibitor is a serine protease inhibitor.
Embodiment 12. The food product of any one of embodiments 7-10, wherein said protease inhibitor is selected from the group consisting of 4-benzenesulfonyl fluoride hydrochloride (AEBSF), nafamostat, secretory leucocyte protease inhibitor (SLPI), serpins, elafin, a host metabolite, a bacterial metabolite, and combinations thereof.
Embodiment 13. The food product of any one of embodiments 7-10, wherein said protease inhibitor inhibits the activity of a protease selected from the group consisting of trypsin-1, trypsin-2, trypsin-3, chymotrypsin like elastase 2A, chymotrypsin like elastase 3B, chymotrypsin, a kallikrein, plasmin, thrombin, and combinations thereof.
Embodiment 14. A composition comprising a microorganism comprising an exogenous nucleic acid encoding a protease inhibitor.
Embodiment 15. The composition of embodiment 14, wherein said microorganism is selected from the group consisting of *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.*
Embodiment 16. The composition of embodiment 14 or embodiment 15, said composition comprising *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.*
Embodiment 17. The composition of any one of embodiments 14-16, wherein said protease inhibitor is a serine protease inhibitor.
Embodiment 18. The composition of any one of embodiments 14-16, wherein said protease inhibitor is selected from the group consisting of secretory leucocyte protease inhibitor (SLPI), serpins, and elafin.
Embodiment 19. The composition of any one of embodiments 14-16, wherein said protease inhibitor inhibits the activity of a protease selected from the group consisting of trypsin-1, trypsin-2, trypsin-3, chymotrypsin like elastase 2A, chymotrypsin like elastase 3B, chymotrypsin, a kallikrein, plasmin, thrombin, and combinations thereof.
Embodiment 20. The composition of any one of embodiments 14-19, wherein said composition is formulated for oral administration.
Embodiment 21. The composition of embodiment 20, wherein said composition is selected from the group consisting of a liquid, a tablet, a capsule, a pill, a powder, a gel, and granules.
Embodiment 22. A method for treating a mammal having irritable bowel syndrome (IBS), wherein said method comprises administering a composition comprising a protease inhibitor to said mammal.
Embodiment 23. The method of embodiment 22, wherein said method comprises identifying said mammal as having said IBS.
Embodiment 24. The method of any one of embodiments 22-23, wherein said mammal is a human.
Embodiment 25. The method of any one of embodiments 22-24, wherein IBS is post-infection IBS (PI-IBS).
Embodiment 26. The method of any one of embodiments 22-25, wherein administering said composition to said mammal is effective to reduce or eliminate a symptom of said IBS in said mammal.
Embodiment 27. The method of embodiments 26, wherein said symptom of IBS is selected from the group consisting of abdominal pain, abdominal cramping, abdominal bloating, excess gas, diarrhea constipation, alternating bouts of diarrhea and constipation, weight loss, rectal bleeding, iron deficiency anemia, unexplained vomiting, difficulty swallowing, and combinations thereof.
Embodiment 28. The method of any one of embodiments 22-24, wherein administering said composition to said mammal is effective to reduce intestinal permeability in the gastrointestinal tract of said mammal.
Embodiment 29. The method of any one of embodiments 22-24, wherein administering said composition to said mammal is effective to reduce a level of proteolytic activity of a protease in the gastrointestinal tract of said mammal.
Embodiment 30. The method of embodiment 29, wherein said protease is selected from the group consisting of trypsin-1, trypsin-2, trypsin-3, chymotrypsin like elastase 2A, chymotrypsin like elastase 3B, chymotrypsin, a kallikrein, plasmin, thrombin, and combinations thereof.
Embodiment 31. The method of embodiment 29 or embodiment 30, wherein the level of proteolytic activity of the protease is measured using zymography.
Embodiment 32. The method of any one of embodiments 22-24, wherein administering said composition to said mammal is effective to increase the level of a protease inhibitor in the gastrointestinal tract of said mammal.
Embodiment 33. The method of embodiment 32, wherein said protease inhibitor is selected from the group consisting of 4-benzenesulfonyl fluoride hydrochloride (AEBSF), nafamostat, secretory leucocyte protease inhibitor (SLPI), serpins, elafin, a host metabolite, a bacterial metabolite, and combinations thereof.
Embodiment 34. A method for treating a mammal having irritable bowel syndrome (IBS), wherein said method comprises administering a composition comprising a microorganism comprising nucleic acid encoding a protease inhibitor to said mammal.
Embodiment 35. The method of embodiment 34, wherein said method comprises identifying said mammal as having said IBS.
Embodiment 36. The method of any one of embodiments 34-35, wherein said mammal is a human.
Embodiment 37. The method of any one of embodiments 34-36, wherein IBS is post-infection IBS (PI-IBS).
Embodiment 38. The method of any one of embodiments 34-37, wherein administering said composition to said mammal is effective to reduce or eliminate a symptom of said IBS in said mammal.
Embodiment 39. The method of embodiment 38, wherein said symptom of IBS is selected from the group consisting of abdominal pain, abdominal cramping, abdominal bloating, excess gas, diarrhea constipation, alternating bouts of diarrhea and constipation, weight loss, rectal bleeding, iron deficiency anemia, unexplained vomiting, difficulty swallowing, and combinations thereof.
Embodiment 40. The method of any one of embodiments 33-37, wherein administering said composition to said mammal is effective to reduce intestinal permeability in the gastrointestinal tract of said mammal.
Embodiment 41. The method of any one of embodiments 34-37, wherein administering said composition to said mammal is effective to reduce a level of proteolytic activity of a protease in the gastrointestinal tract of said mammal.
Embodiment 42. The method of embodiment 41, wherein said protease is selected from the group consisting of trypsin-1, trypsin-2, trypsin-3, chymotrypsin like elastase 2A, chymotrypsin like elastase 3B, chymotrypsin, a kallikrein, plasmin, thrombin, and combinations thereof.
Embodiment 43. The method of embodiment 41 or embodiment 42, wherein the level of proteolytic activity of the protease is measured using zymography.
Embodiment 44. The method of any one of embodiments 34-37, wherein administering said composition to said mammal is effective to increase the level of a protease inhibitor in the gastrointestinal tract of said mammal.
Embodiment 45. The method of embodiment 44, wherein said protease inhibitor is selected from the group consisting of secretory leucocyte protease inhibitor (SLPI), serpins, and elafin.
Embodiment 46. The method of any one of embodiments 34-45, wherein said microorganism comprising said nucleic acid encoding said protease inhibitor is selected from the group consisting of *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.*
Embodiment 47. A composition comprising a microorganism comprising an exogenous nucleic acid encoding a glucuronidase.
Embodiment 48. The composition of embodiment 47, wherein said microorganism is selected from the group consisting of *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.*
Embodiment 49. The composition of embodiment 47 or embodiment 48, said composition comprising *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.*
Embodiment 50. The composition of any one of embodiments 47-49, wherein said glucuronidase is a beta-glucuronidase.
Embodiment 51. The composition of any one of embodiments 47-50, wherein said glucuronidase converts bilirubin to unconjugated bilirubin.
Embodiment 52. The composition of any one of embodiments 47-51, wherein said composition is formulated for oral administration.
Embodiment 53. The composition of embodiments 52, wherein said composition is selected from the group consisting of a liquid, a tablet, a capsule, a pill, a powder, a gel, and granules.
54. A composition comprising one or more microorganisms comprising an exogenous nucleic acid encoding a glucuronidase.
55. The composition of claim 54, wherein said one or more microorganisms are selected from the group consisting of *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.*
56. The composition of claim 54 or claim 55, said composition comprising *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.*
57. The composition of any one of claims 54-56, wherein said glucuronidase is a beta-glucuronidase.
58. The composition of any one of claims 54-57, wherein said glucuronidase converts bilirubin to unconjugated bilirubin.
59. The composition of any one of claims 54-58, wherein said composition is formulated for oral administration.
60. The composition of claim 59, wherein said composition is selected from the group consisting of a liquid, a tablet, a capsule, a pill, a powder, a gel, and granules.
61. A method for treating a mammal having irritable bowel syndrome (IBS), wherein said method comprises administering the composition of any one of embodiments 14-21 and 54-60.
62. The use of the composition of any one of embodiments 14-21 and 54-60 to treat a mammal having irritable bowel syndrome (IBS).
63. The use of the composition of any one of embodiments 14-21 and 54-60 in the manufacture of a medicament to treat a mammal having irritable bowel syndrome (IBS).
64. The method of claim 61 or the use of any one of claims 62-63, wherein said mammal is a human.
65. The method of claim 61 or the use of any one of claims 62-63, wherein IBS is post-infection IBS (PI-IBS).
66. The method of claim 61 or the use of any one of claims 62-63, wherein said composition is effective to reduce or eliminate a symptom of said IBS in said mammal.
67. The method or use of claim 66, wherein said symptom of IBS is selected from the group consisting of abdominal pain, abdominal cramping, abdominal bloating, excess gas, diarrhea constipation, alternating bouts of diarrhea and constipation, weight loss, rectal bleeding, iron deficiency anemia, unexplained vomiting, difficulty swallowing, and combinations thereof.
68. The method of claim 61 or the use of any one of claims 62-63, wherein said composition is effective to reduce intestinal permeability in the gastrointestinal tract of said mammal.
69. The method of claim 61 or the use of any one of claims 62-63, wherein said composition is effective to reduce a level of proteolytic activity of a protease in the gastrointestinal tract of said mammal.
70. The method or use of claim 69, wherein said protease is selected from the group consisting of trypsin-1, trypsin-2, trypsin-3, chymotrypsin like elastase 2A, chymotrypsin like elastase 3B, chymotrypsin, a kallikrein, plasmin, thrombin, and combinations thereof.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### EMBODIMENTS

Although the present invention is defined in the claims, it should be understood that the present invention can also (alternatively) be defined in accordance with the following embodiments:
1. A composition comprising a microorganism comprising an exogenous nucleic acid encoding a protease inhibitor.
2. The composition of embodiment 1, wherein said microorganism is selected from the group consisting of *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii,* and *Roseburia hominis, .*
3. The composition of embodiment 1 or embodiment 2, said composition comprising *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii,* and *Roseburia hominis.*
4. The composition of any one of embodiments 1-3, wherein said protease inhibitor is a serine protease inhibitor.
5. The composition of any one of embodiments 1-3, wherein said protease inhibitor is selected from the group consisting of secretory leucocyte protease inhibitor (SLPI), serpins, and elafin.
6. The composition of any one of embodiments 1-3, wherein said protease inhibitor inhibits the activity of a protease selected from the group consisting of trypsin-1, trypsin-2, trypsin-3, chymotrypsin like elastase 2A, chymotrypsin like elastase 3B, chymotrypsin, a kallikrein, plasmin, thrombin, and combinations thereof.
7. The composition of any one of embodiments 1-6, wherein said composition is formulated for oral administration.
8. The composition of embodiment 7, wherein said composition is selected from the group consisting of a liquid, a tablet, a capsule, a pill, a powder, a gel, and granules.
9. The composition of any one of embodiments 1-8, wherein said composition comprises *Prevotella copri* and *Bacteroides ovatus.*
10. A composition comprising one or more microorganisms comprising an exogenous nucleic acid encoding a glucuronidase.
11. The composition of embodiment 10, wherein said one or more microorganisms are selected from the group consisting of *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.*
12. The composition of embodiment 10 or embodiment 11, said composition comprising *Alistipes putredinis, Ruminococcus bromii, Alistipes finegoldi, Alistipes shahii, Collinsella aerofaciens, Alistipes onderdonkii, Eubacterium siraeum, Odoribacter splanchnicus, Adlercreutzia equolifaciens, Barnesiella intestinihominis, Parabacteroides goldsteinii, Roseburia hominis, Prevotella copri,* and *Bacteroides ovatus.*
13. The composition of any one of embodiments 10-12, wherein said glucuronidase is a beta-glucuronidase.
14. The composition of any one of embodiments 10-13, wherein said glucuronidase converts conjugated bilirubin to unconjugated bilirubin.
15. The composition of any one of embodiments 10-14, wherein said composition is formulated for oral administration.
16. The composition of embodiment 15, wherein said composition is selected from the group consisting of a liquid, a tablet, a capsule, a pill, a powder, a gel, and granules.
17. A method for treating a mammal having irritable bowel syndrome (IBS), wherein said method comprises administering the composition of any one of embodiments 1-16.
18. The use of the composition of any one of embodiments 1-16 to treat a mammal having irritable bowel syndrome (IBS).
19. The use of the composition of any one of embodiments 1-16 in the manufacture of a medicament to treat a mammal having irritable bowel syndrome (IBS).
20. The method of embodiment 17 or the use of any one of embodiments 18-19, wherein said mammal is a human.
21. The method of embodiment 17 or the use of any one of embodiments 18-19, wherein IBS is post-infection IBS (PI-IBS).
22. The method of embodiment 17 or the use of any one of embodiments 18-19, wherein said composition is effective to reduce or eliminate a symptom of said IBS in said mammal.
23. The method or use of embodiment 22, wherein said symptom of IBS is selected from the group consisting of abdominal pain, abdominal cramping, abdominal bloating, excess gas, diarrhea constipation, alternating bouts of diarrhea and constipation, weight loss, rectal bleeding, iron deficiency anemia, unexplained vomiting, difficulty swallowing, and combinations thereof.
24. The method of embodiment 17 or the use of any one of embodiments 18-19, wherein said composition is effective to reduce intestinal permeability in the gastrointestinal tract of said mammal.
25. The method of embodiment 17 or the use of any one of embodiments 18-19, wherein said composition is effective to reduce a level of proteolytic activity of a protease in the gastrointestinal tract of said mammal.
26. The method or use of embodiment 25, wherein said protease is selected from the group consisting of trypsin-1, trypsin-2, trypsin-3, chymotrypsin like elastase 2A, chymotrypsin like elastase 3B, chymotrypsin, a kallikrein, plasmin, thrombin, and combinations thereof.

## Claims

1. A nutritional supplement comprising *Bacteroides ovatus,* wherein said nutritional supplement is selected from the group consisting of a tablet, a capsule, and a pill.

2. The nutritional supplement of claim 1, wherein said microorganism is encapsulated to be released in the intestine of a mammal.

3. The nutritional supplement of claim 1 or claim 2, wherein said nutritional supplement is a capsule.

4. The nutritional supplement of any one of claims 1-3, wherein said nutritional supplement comprises a protease inhibitor.

5. The nutritional supplement of claim 4, wherein said protease inhibitor is selected from the group consisting of 4-benzenesulfonyl fluoride hydrochloride (AEBSF), nafamostat, secretory leucocyte protease inhibitor (SLPI), serpins, elafin, a host metabolite, and a bacterial metabolite.

6. The nutritional supplement of claim 4 or claim 5, wherein said protease inhibitor inhibits the activity of a protease selected from the group consisting of trypsin-1, trypsin-2, trypsin-3, chymotrypsin like elastase 2A, chymotrypsin like elastase 3B, chymotrypsin, a kallikrein, plasmin, and thrombin.

7. A composition for use in a method for treating a mammal having Irritable Bowel Syndrome (IBS),
wherein said method comprises administering, to said mammal, said composition, and
wherein said composition comprises a microorganism containing nucleic acid encoding a protease inhibitor, wherein said microorganism is *Bacteroides ovatus.*

8. The composition for use of claim 7, wherein said mammal is a human; and/or wherein said IBS is post-infection IBS (PI-IBS).

9. The composition for use of any one of claims 7-8, wherein said composition is effective to reduce or eliminate a symptom of said IBS in said mammal, optionally wherein said symptom of IBS is selected from the group consisting of abdominal pain, abdominal cramping, abdominal bloating, excess gas, diarrhea constipation, alternating bouts of diarrhea and constipation, weight loss, rectal bleeding, iron deficiency anemia, unexplained vomiting, difficulty swallowing, and combinations thereof.

10. The composition for use of any one of claims 7-9, wherein said nucleic acid encoding a protease inhibitor is an endogenous nucleic acid.

11. The composition for use of any one of claims 7-9, wherein said nucleic acid encoding a protease inhibitor is an exogenous nucleic acid.

12. The composition for use of any one of claims 7-11, wherein the protease inhibitor is selected from the group consisting of secretory leucocyte protease inhibitor (SLPI), serpins, elafin, a host metabolite, and a bacterial metabolite.

13. The composition for use of any one of claims 7-12, wherein said composition is effective to reduce intestinal permeability in the gastrointestinal tract of said mammal.

14. The composition for use of any one of claims 7-13, wherein said composition is effective to reduce a level of proteolytic activity of a protease in the gastrointestinal tract of said mammal, optionally wherein said protease is selected from the group consisting of trypsin-1, trypsin-2, trypsin-3, chymotrypsin like elastase 2A, chymotrypsin like elastase 3B, chymotrypsin, a kallikrein, plasmin, and thrombin.

15. The composition for use of any one of claims 7-14, wherein said microorganism contains nucleic acid encoding a glucuronidase, optionally wherein said glucuronidase is a beta-glucuronidase.
